(19) Europäisches Patentamt
European Patent Office
Office européen des brevets

(11) Numéro de publication: **0 251 937**
**B1**

# (12) FASCICULE DE BREVET EUROPEEN

(45) Date de publication du fascicule du brevet: **30.01.91**

(21) Numéro de dépôt: **87401562.1**

(22) Date de dépôt: **03.07.87**

(51) Int. Cl.$^5$: **C 07 D 237/36,**
C 07 D 237/26,
C 07 D 413/12, A 61 K 31/50,
C 07 D 403/12

## (54) Nouveaux dérivés tricycliques agonistes des récepteurs cholinergiques et médicaments en contenant.

(30) Priorité: **03.07.86 FR 8609681**

(43) Date de publication de la demande:
**07.01.88 Bulletin 88/01**

(45) Mention de la délivrance du brevet:
**30.01.91 Bulletin 91/05**

(84) Etats contractants désignés:
**AT BE CH DE ES FR GB GR IT LI LU NL SE**

(56) Documents cités:
**EP-A-0 072 299**

(73) Titulaire: **SANOFI**
**40, Avenue George V**
**F-75008 Paris (FR)**

(72) Inventeur: **Biziere, Kathleen**
**6, Lotissements Rayons d'Oc**
**F-34170 Clapiers (FR)**
Inventeur: **Wermuth, Camille Georges**
**3, rue de la Côte d'Azur**
**F-67100 Strasbourg (FR)**
Inventeur: **Worms, Paul**
**10, rue Devois**
**F-34980 Saint Gely Du Fesc (FR)**
Inventeur: **Bourguignon, Jean-Jacques**
**2, rue du Feld Vasser**
**F-67150 Hipsheim (FR)**

(74) Mandataire: **Gillard, Marie-Louise et al**
**Cabinet Beau de Loménie 55, Rue d'Amsterdam**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 251 937 B1

Courier Press, Leamington Spa, England.

## Description

Les démences séniles et en particulier les démences de type Alzeheimer sont des affections graves dont la fréquence tend à augmenter en fonction de l'augmentation de la longévité de la population.

Les études entreprises par différents auteurs, ont mis en évidence dans la maladie d'Alzheimer l'existence d'un déficit spécifique des marqueurs cholinergiques corticaux provoquant des troubles graves des fonctions supérieures.

Les résultats obtenus par utilisation d'agonistes muscariniques pour le traitement des démences séniles se sont montrés encourageants. Toutefois les agonistes muscariniques n'existent qu'en petit nombre et se sont avérés de maniement difficile chez l'homme.

Par suite la recherche d'agonistes muscariniques post-synaptiques comme traitement de la maladie d'Alzheimer s'avère aujourd'hui tout à fait souhaitable.

L'intérêt de disposer d'agonisters muscariniques centraux sélectifs pour remédier au déficit cholinergique dans la maladie d'Alzheimer a été mentionné notamment dans ISI Atlas of Science: Pharmacology (1987), p. 98 à 100.

C'est à ce problème que la présente invention tente d'apporter une solution par de nouveaux produits actifs sélectivement sur les récepteurs muscariniques centraux $M_1$.

Selon un premier aspect, la présente invention a pour objet des composés tricycliques nouveaux répondant à la formule générale:

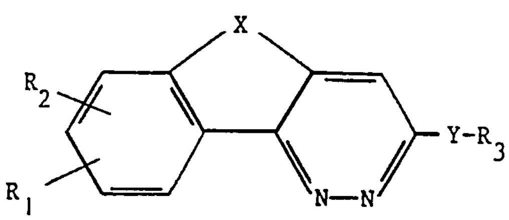

(I)

dans laquelle:

X représente un groupe $(CH_2)_n$ avec n représentant un entier égal à 2, 3 ou 4 ou encore un groupe vinylène ou un groupe méthylevinylène;

$R_1$ et $R_2$ considérés indépendamment représentent l'hydrogène ou un substituant occupant l'une des positions libres du cycle benzénique et choisi parmi les halogènes, un groupe alkyle inférieur, un groupe alcoxy inférieur, un groupe hydroxy, un groupe thiol, un groupe nitro, un groupe amino;

Y représente l'oxygène, le soufre ou un groupe —NH—;

$R_3$ represente:

un groupe

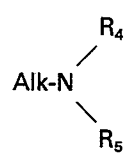 dans lequel:

Alk représente un groupe alkylène droit ou ramifié ayant de 2 à 5 atomes de carbone,

$R_4$ et $R_5$ représentent chacun indépendamment l'hydrogène, un groupe alkyle inférieur, un groupe hydroxyalkyle inférieur ou encore $R_4$ et $R_5$ constituent avec l'atome d'azote auquel ils sont liés un groupe choisi parmi les groupes pyrrolidinyl-1, pipéridino, morpholino, pipérazinyl-1, oxo-2 morpholino;

un groupe

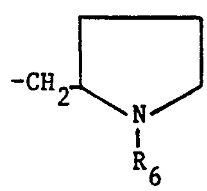

où $R_6$ représente un groupe alkyle inférieur ayant 1 à 4 atomes de carbone;

un groupe

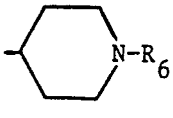

où $R_6$ est comme défini ci-dessus; ainsi que leurs sels d'addition avec les acides minéraux ou organiques pharmaceutiquement acceptables.

Selon un second aspect l'invention a pour objet un procédé de préparation des composés de formule (I) qui peut être représenté par le schéma suivant:

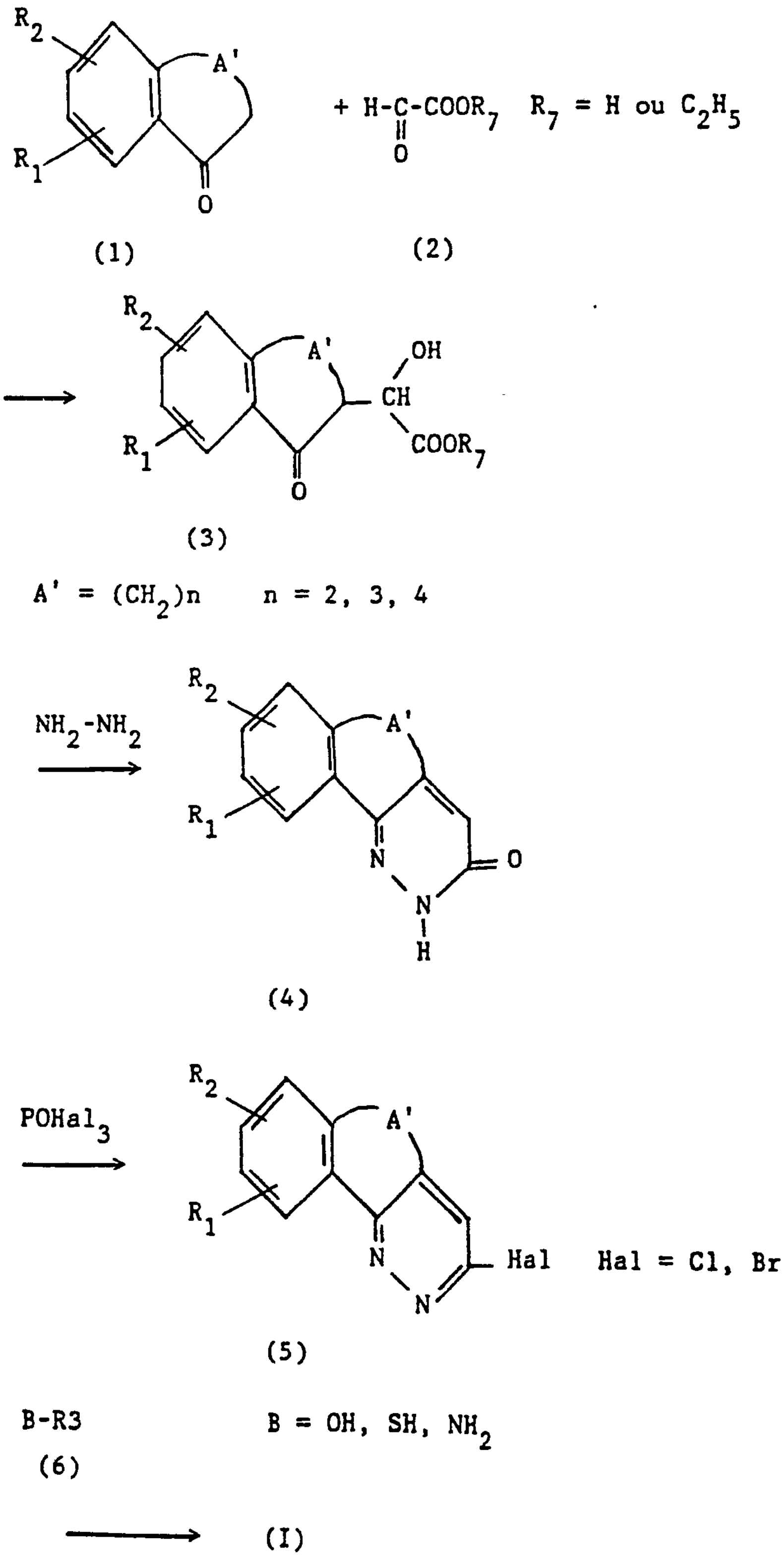


Par action du glyoxylate d'éthyle ou de l'acide glyoxylique (2) sur une benzocyclanone (1) à température comprise ente 60 et 150°C, on forme l'hydroxyester ou l'hydroxyacide (3). Celui-ci contient le

plus souvent de 5 à 15% du produit de déshydratation correspondant (ester acrylique). On peut le purifier par chromatographie ou encore utiliser directement le produit brut pour l'étape suivante.

Le produit (3) par chauffage avec de l'hydrate d'hydrazine fournit la pyridazone (4). On opère soit avec un large excès d'hydrate d'hydrazine, soit au sein d'un solvant choisi parmi les solvants hydroxylés et notamment le n-butanol ou encore le toluène.

Dans la plupart des cas on obtient directement la pyridazone (4) par action de l'hydrate d'hydrazine comme indiqué ci-dessus.

Dans quelques cas, l'hydroxy-4 pyridazone-3 formée intermédiairement ne se déshydrate pas spontanément lors de la réaction avec l'hydrate d'hydrazine. Dans ce cas, il faut la déshydrater par chauffage à 140°C avec un agent de déshydratation tel que l'acide polyphosphorique.

La pyridazone (4) traitée au reflux par un excés d'oxychlorure ou d'oxybromure de phosphore conduit au dérivé halogéné (5).

Enfin par chauffage du dérivé halogéné (5) avec le dérivé (6) au sein d'un solvant convenable on obtient le composé (I).

Le solvant peut être soit un solvant hydroxylé tel que le n-butanol, soit du diméthylformamide ou encore il peut être constitué par un excès du dérivé (6).

Lorsque la réaction de substitution de dérivé chloré s'avère lente, elle peut être facilitée par addition de chlorure d'ammonium si B est $NH_2$ ou par addition d'hydrure de sodium lorsque B représente OH ou SH.

Eventuellement, les composés (I) ainsi obtenus peuvent être salifiés par un procédé connu.

Lorsque le composé (I) comporte un substituant $R_1$ ou $R_2$ susceptible de réagir au cours d'une ou plusieurs étapes de la synthèse, ils doivent être bloqués à l'aide d'un réactif convenable qui permittre de les régénérer en fin de synthèse.

Selon une variante du procédé les composés (I) monosubstitués sur le noyau benzénique, en position méta par rapport à la liaison directe entre le cycle benzénique et le cycle pyridazinique, peuvent être préparés à partir du dérivé chloré (5) ($R_1=R_2=H$) par nitration qui conduit au dérivé nitré correspondant (5) ($R_1=NO_2$, $R_2=H$).

Celui-ci est transformé en composé (I) correspondant ainsi qu'indiqué précédemment.

A partir des dérivés (I) nitrés ainsi obtenus on peut par une ou plusieurs réactions connues transformer le dérivé nitré en composés (I) où $R_1$ représente divers substituants.

Ainsi par réduction catalytique, on obtient les composés (I) $R_1=NH_2$, $R_2=H$ et à partir de ceux-ci on peut par diazotation suivie de décomposition du sel de diazonium en présence de réactifs convenables obtenir les composés (I) où $R_1$ est halogène, hydroxy, . . . . .

La nitration du dérivé chloré peut, selon une variante, être suivie directement d'une ou plusieurs réactions de transformation du groupe nitro en un autre substituant. A partir des composés chlorés ainsi obtenus (de formule (c) dans laquelle $R_1$, en para de X, est autre que l'hydrogène alors que $R_2$=H), on obtient les composés (I) correspondants comme indiqué précédemment (action de B—$R_3$).

Selon une seconde variante du procédé applicable aux composés (I) où Y est un atome de soufre, on peut transformer la pyridazone(4) en thione(7) par action de pentasulfure de phosphore

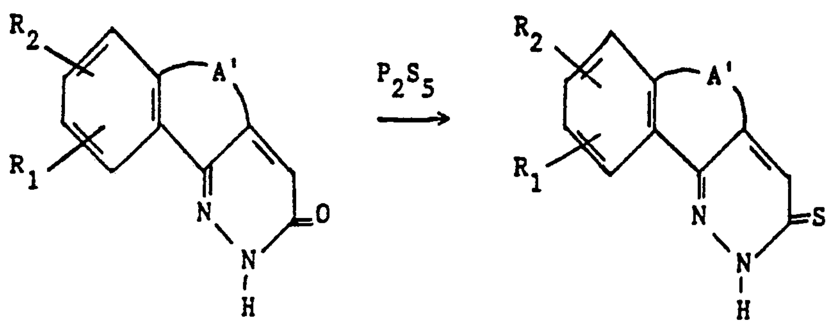


(4) (7)

On opère par chauffage à 80—100°C dans un solvant convenable tel que l'acétonitrile, l'éther diméthylique de l'éthylène glycol ou un mélange des deux ou encore la pyridine, le toluène ou le xylène, de la pyridazone avec un excès de pentasulfure de phosphore en présence de bicarbonate de sodium.

On substitue ensuite la thione(7) par action d'un halogènure Hal-$R_3$ soit en solution dans un alcool en présence de l'alcoolate de sodium correspondant, soit dans le diméthylformamide pour obtenir le composé (I) qu'on purifie par chromatographie.

Les composés où A' représente un groupe vinylène, sont obtenus conjointement avec les composés où A' représente un groupe éthylène par chauffage du dérivé chloré (5) avec un excés de dérivé (6) en présence de chlorure d'ammonium.

Par chromatographie, on sépare les 2 constituents du mélange ainsi obtenu.

Les composés (I) où A' représente un groupe méthyl vinylène sont obtenus à partir des pyridazones (4)

où A′ représente un groupe méthyl éthylène. L'action de l'oxychlorure de phosphore sur ces pyridazones, provoque à la fois la formation de la chloro-3 pyridazine et la déshydrogénation du groupe méthyl éthylène en groupe méthylvinylène.

Enfin, les composés (I) où $R_3$ représente un groupe:

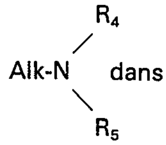 dans lequel 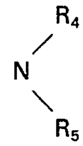

désigne un groupe oxo-2 morpholine, peuvent être préparés à partir des composés (I) correspondants dans lesquels

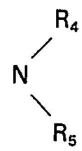

représente un groupe $NHCH_2CH_2OH$ par substitution de l'azote par le chloracétate d'éthyle et cyclisation de l'hydroxyester ainsi obtenu.

Les cycloalkanones de départ de formule (1) sont connues ou peuvent être préparées par des procédés connus et notamment par cyclisation interne des acides:

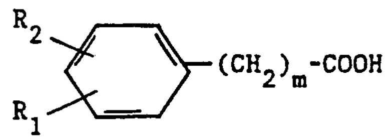 (8)

où m est égal à n+1 en présence d'acide sulfurique ou d'acide polyphosphorique. Les acides (8) sont eux mêmes connus ou peuvent être préparés par des procédés connus par exemple par réduction des oxo acides correspondants:

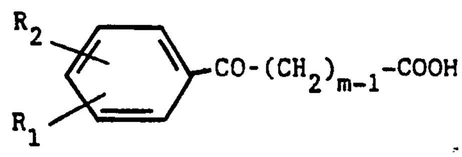

Les exemples suivants sont fournis pour illustrer l'invention

Exemple 1

Dichlorhydrate de (morpholino-2 ethylamino)-3 dihydro-6,7 5H benzocyclohepta [5,6-c] pyridazine (SR 95 639A)

(I) $X = (CH_2)_3$ ; $R_1 = R_2 = H$ ; $Y = NH$

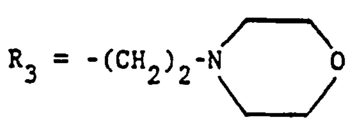

A) (oxo-5 tétrahydro-6,7,8,9 5H-benzocycloheptenyl-6) glycolate d'éthyle

On chauffe à 135°C pendant 24 heures, le mélange de 48 g de tétrahydro-6,7,8,9 5H-benzocyclohepténone-5 et 33,6 g de glyoxylate d'éthyle. Après refroidissement, on purifie le mélange réactionnel par passage sur une colonne de silica. En éluant avec le mélange cyclohexane-acétate d'éthyle 95/5 (vol/vol) on obtient le produit attendu sous forme d'une huile jaune.

Poids 32g; Rendement 41%.

B) Tétrahydro-2,3,6,7 5H-benzocyclohepta [5,6-c] pyridazone-3

On chauffe au reflux la solution de 15 g du produit obtenu ci-dessus dans 250 ml de n-butanol. On

ajoute goutte à goutte à la solution, 4,25 g d'hydrate d'hydrazine et poursuit le reflux pendant 15 heures. On évapore le solvant sous vide et recristallise le résidu dans l'éthanol à 95°.

F: 235°C.

C) Chloro-3 dihydro-6,7 5H-benzocyclohepta [5,6-c] pyridazine.

On chauffe au reflux pendant 5 heures le mélange de 5 g de la pyridazone préparée ci-dessus et 100 ml d'oxychlorure de phosphore.

On verse le mélange réactionnel sur de la glace et alcalinise par une solution aqueuse de soude à 20%. On essore le solide quie se sépare, lave avec de l'eau et sèche à l'air. On recristallise dans le méthanol.

F: 159°C.

D) SR 95639 A

On chauffe à 135°C sous argon pendant 6 heures, le mélange de 4,6 g de dérivé chloré préparé ci-dessus, 10,4 g de N-(amino-2 éthyl) morpholine et 1,06 g de chlorure d-ammonium.

On verse le mélange réactionnel dans l'eau et essore le solide qui se sépare. On recristallise dans l'éthanol à 95°.

F: 105°C; Poids: 4 g; Rendement: 62,5%.

Dichlorhydrate

A la solution de 4 g de base dans le minimum d'isopropanol chaud, on ajoute 2,08 ml d'acide chlorhydrique concentré.

Par addition d'éther, le dichlorhydrate précipite. On l'essore et recristallise dans l'éthanol à 95°.

F: 112°; Poids 5 g; Rendement 91%.

Analyse pour $C_{19}H_{24}$ NO, 2 HCl, 2,5 $H_2O$. (443,38).

| | C | H | N |
|---|---|---|---|
| Calculé: | 51,46 | 7,05 | 12,64 |
| Trouvé: | 51,65 | 7.23 | 12,78 |

Exemples 2 à 8

A partir du derivé chloré de l'exemple 1—C et en opérant comme dans l'exemple 1—D mais en faisant varier le dérivé aminé utilisé, on obtient les produits de formule (I) $R_1=R_2=H$, $X=(CH_2)_3$, Y=NH réunis dans le tableau ci-dessous.

Dans le case où la base ne cristallise pas, on l'extrait avec de l'acétate d'éthyle et forme le sel après évaporation du solvant.

TABLEAU 1

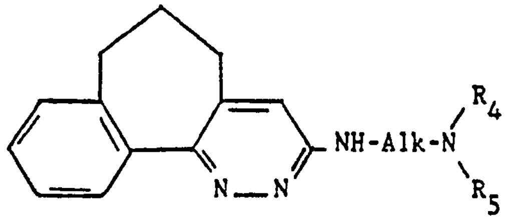

| N° Exemple | N° Code SR | Alk | 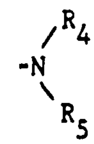 | Sel F : °C |
|---|---|---|---|---|
| 2 | 95640 A | $(CH_2)_2$ | 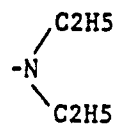 | Dichlorhydrate: 2,5 $H_2O$ 111°C |
| 3 | 95817 A | $(CH_2)_3$ | 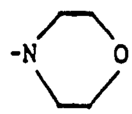 | Dichlorhydrate: 222°C |
| 4 | 95818 A | $(CH_2)_3$ | 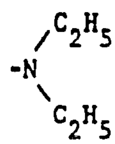 | Dichlorhydrate: 198°C |
| 5 | 95819 A | $(CH_2)_2$ | $-NH-CH_2$<br>$CH_2OH$ | Dichlorhydrate: 190°C |
| 6 | 95824 A | $(CH_2)_2$ | 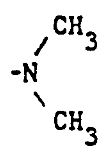 | Dichlorhydrate: 0,5 $H_2O$ 234-236°C |
| 7 | 44694 A | $(CH_2)_2$ | 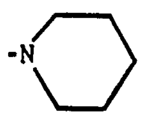 | Dichlorhydrate: 235-238°C |
| 8 | 95883 A | $(CH_2)_2$ | 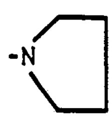 | Dichlorhydrate: 137-139°C |

Exemple 9

Dichlorhydrate de fluoro-9 (morpholino-2 éthylamino)-3 dihydro-6,7 5H benzocyclohepta [5,6-c] pyridazine (SR 96 108 A)

(I) $X = (CH_2)_3$; $R_1 \div 9\text{-F}$; $R_2 = H$; $Y = NH$

$R_3 = (CH_2)_2-$ 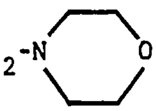

On opère comme dans l'exemple 1 en utilisant comme produit de départ la fluoro-2 tétrahydro-6,7,8,9 5H benzocycloheptenone-5 préparée selon Journal of Organic Chemistry 1962, *27,* 70—76.

De la même façon on obtient le produit attendu.

Base F: 134°C;

Dichlorhydrate F: 182—183°C.

Exemple 10

Dichlorhydrate de (diéthylamino-2 éthylamino)-3 diméthyl-9,10 dihydro-6,7 5H benzocyclohepta [5,6-c] pyridazine (SR 44663 A)

(I) $X=(CH_2)_3$; $R_1=9\text{-}CH_3$; $R_2=10\text{-}CH_3$;

Y=NH; R3= 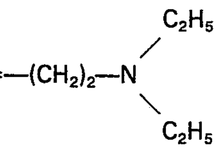

A) Diméthyl-9,10 tétrahydro-2,3,6,7 5H benzocyclohepta [5,6-c] pyridazone-3

On chauffe à 75°C pendant 15 heures le mélange de 27,15 g de diméthyl-2,3 tétrahydro-6,7,8,9 5H benzocycloheptenone-5 et 13,2 g d'hydrate de l'acide glyoxylique. Après refroidissement on reprend le mélange réactionnel dans l'eau et le chlorure de méthyléne et alcalinise par addition de carbonate de potassium.

On sépare la phase aqueuse qu'on extrait une seconde fois avec du chlorure de méthylène. La phase aqueuse est refroidie et acidifiée par addition d'acide chlorhydrique concentré. On extrait 3 fois avec du chlorure de méthylène et sèche la solution organique puis on évapore le solvant à siccité. Le produit cristallise et est utilisé tel quel pour l'étape suivants; Poids 24 g.

On dissout le produit dans 140 ml de n-butanol et ajoute 7 ml d'hydrate d'hydrazine. On chauffe 15 heures au reflux puis refroidit le mélange réactionnel à 4°C. On essore le solide qu'on lave avec un peu d'isopropanol froid puis avec de l'éther.

Poids: 13,5 g; F: 270°C.

B) Chloro-3 diméthyl-9,10 dihydro-6,7 5H benzocyclohepta [5,6-c] pyridazine

On chauffe au reflux pendant 4 heures le mélange de 5 g de la pyridazone obtenue en A) et 9,3 ml d'oxychlorure de phosphore dans 26 ml d'acétonitrile.

On verse le mélange réactionnel dans 400 ml d'eau et amène le pH à 8 par addition d'ammoniaque concentré. On essore les cristaux qu'on lave avec de l'eau et sèche.

Poids: 5,15 g; F: 183—4°C.

C) SR 44663 A

On chauffe au reflux pendant 24 heures le mélange de 5 g de dérivé chloré obtenu ci-dessus et 12,5 ml de diéthylamino-2 éthylamine dans 100 ml de n-butanol.

On élimine le solvant et l'excès d'amine par évaporation sous vide puis reprend le résidu dans le chlorure de méthylène. On lave la solution avec de l'eau puis sèche sur sulfate de magnésium.

Aprés purification par chromatographie sur silice la produit cristallise sous forme de base.

F: 88—9°C.

Dichlorhydrate

On prépare le dichlorhydrate par dissolution de la base dans le minimum d'isopropanol chaud et addition d'ether chlorhydrique.

Après séchage sous vide, on obtient 3,35 g de dichlorhydrate.

F: 140—145°C, hygroscopique.

Exemple 11

Dichlorhydrate de dichloro-9,10 (diéthylamino-2 éthylamino)-3 dihydro-6,7 5H benzocyclohepta [5,6-c] pyridazine (SR 44 695 A)

(I) $X=(CH_2)_3$; $R_1=9\text{-Cl}$; $R_2=10\text{-Cl}$; Y=NH

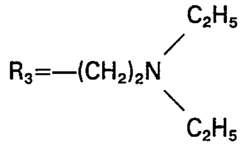

En opérant comme dans l'exemple 10 à partir de dichloro-2,3 tétrahydro-6,7,8,9 5H benzocyclohepténone-5, on obtient successivement:

la dichloro-9,10 tétrahydro-2,3,6,7 5H benzocyclohepta [5,6-c] pyridazone-3, F > 250°C;

la trichloro-3,9,10 dihydro-6,7 5H benzocyclohepta [5,6-c] pyridazine, F: 185—186°C;

SR 44695 A: Base F: 100—101°C

Dichlorohydrate: F: 100—105°C.

Exemples 12 à 16

En opérant comme dans l'exemple 10 à partir des dérivés chlorés en 3 des exemples 10 et 11 mais en faisant varier l'amine utilisée, on obtient les composés de formule (I) réunis dans le tableau 2.

TABLEAU 2

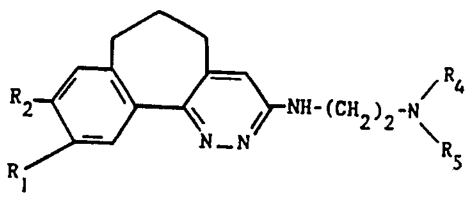

| N° Exemple | N° Code SR | $R_1$ | $R_2$ | 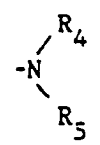 | Sel ou base: F°C |
|---|---|---|---|---|---|
| 12 | 44650 A | $CH_3$ | $CH_3$ | 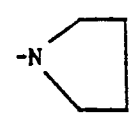 | Dichlorhydrate 140-144°C (hygroscopique) |
| 13 | 44664 A | $CH_3$ | $CH_3$ | 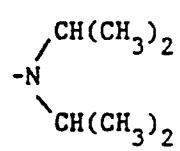 | Base 128-129°C |
| 14 | 44696 A | Cl | Cl | 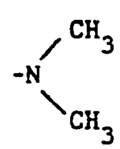 | Base 102-103°C |
| 15 | 44705 A | Cl | Cl | 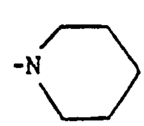 | Base 177-179°C |
| 16 | 44706 A | CH3 | CH3 | 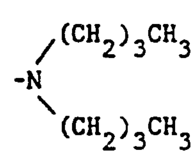 | Dichlorhydrate 156-160°C |

Exemple 17

Dichlorohydrate de [(éthyl-1 pyrrolidinyl-2) méthylamino]-3 dihydro-6,7 5H benzocyclohepta [5,6-c] pyridazine (SR 95 879 A)

$$(I) \quad X = (CH_2)_3 \; ; \; R_1 = R_2 = H \; ; \; Y = NH$$

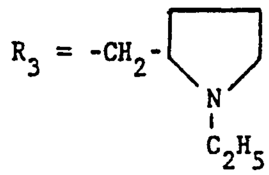

On opère comme dans l'exemple 1D) à partir du dérivé chloré de l'exemple 1C) et d'aminométhyl-2 éthyl-1 pyrrolidine.

De la même façon on obtient le produit attendu isolé sous forme de dichlorhydrate.

F: 158°C.

Exemple 18

Dichlorhydrate de [(méthyl-1 pipéridinyl-4) amino] dihydro-6,7 5H benzocyclohepta [5,6-c] pyridazine (SR 96093 A)

On opère comme dans l'exemple 1D) à partir du dérivé chloré obtenu à l'exemple 1C) et d'amino-4 méthyl-1 pipéridine.

De la même façon, on obtient le produit attendu isolé sous forme de dichlorhydrate.

F: 255°C.

Exemple 19

Dichlorohydrate de diméthyl-10,11 (pipéridino-2 éthylamino)-3 tétrahydro-5,6,7,8 benzocycloocta (5,6-c] pyridazine (SR 44704 A)

$$(I) \quad X = (CH_2)4 \; ; \; R_1 = 10\text{-}CH_3 \; ; \; R_2 = 11\text{-}CH_3 \; ; \; Y = NH$$

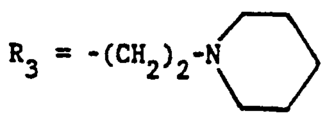

A) Diméthyl-10,11 hexahydro-2,3,5,6,7,8 benzocycloocta [5,6-c] pyridazone-3

On chauffe à 80°C pendant 15 heures le mélange de 13 g de diméthyl-2,3 hexahydro-5,6,7,8,9,10 benzocycloocténone-5 et 5,85 g d'acide glyoxylique monohydrate.

On traite comme indiqué à l'exemple 10-A pour obtenir 17,5 g de produit solide. On reprend ce solide dans 70 ml de n-butanol et ajoute 5 ml d'hydrate d'hydrazine. On porte au reflux pendant 15 heures et refroidit. On essore le solide qui s'est séparé.

Le spectre de RMN montre qu'il s'agit du composé hydroxylé en 4 qui ne s'est pas déshydraté. On reprend donc le solide dans l'acide polyphosphorique (12 ml par gramme de produit) et chauffe à 140°C. On suit l'avancement de la réaction par CCM (éluant $CH_2Cl_2$—MeOH, 95/5 vol/vol). Lorsque le composé hydroxylé a entièrement disparu, on verse le mélange réactionnel dans l'eau glacée. On essore le solide qu'on lave avec de l'eau et sèche à l'étuve sous vide.

B (Chloro-3 diméthyl-10,11 tétrahydro-5,6,7,8 benzocycloocta [5,6-c] pyridazine

On opère comme dans l'exemple 10B) à partir du produit obtenu ci-dessus.

F: 132—134°C.

C) SR 44704 A

On chauffe au reflux pendant 48 heures le mélange de 4,5 g du dérivé chloré obtenu ci-dessus et 12,5 ml de pipéridino-2 éthylamine dans 75 ml de n-butanol.

On évapore le solvant sous vide et reprend le résidu dans l'acétate d'éthyle. On lave la solution avec de l'eau et sèche sur sulfate de magnésium.

On purifie le produit sur colonne de silice. En éluant avec un mélange chlorure de méthylène-méthanol-ammoniaque 80/20/1 vol/vol, on obtient le produit attendu.

F: 115°C.

Dichlorhydrate

On dissout la base dans l'éther et précipite le chlorhydrate par l'acide chlorhydrique.

F: 215°C.

Exemple 20

Dichlorhydrate de (diéthylamino-2 éthylamino)-3 diméthyl-10,11 tétrahydro-5,6,7,8 benzocycloocta [5,6-c] pyridazine (SR 44703 A)

On opère comme dans l'exemple 19C) à partir du dérivé chloré de l'exemple 19B) et de diéthylamino-2 éthylamine.

De la même façon on obtient le dichlorhydrate.

F: 85—88°C.

Exemple 21

Dichlorhydrate de (diéthylamino-2-éthylamino)-3 nitro-10 dihydro-6,7 5H-benzocyclohepta [5,6-c] pyridazine (SR 95777 A)

(I) $X=(CH_2)_3$; $R_1=10\text{-}NO_2$; $R_2=H$; Y=NH

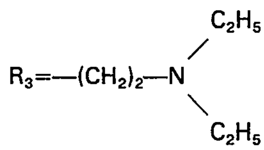

A) Chloro-3 nitro-10 dihydro-6,7 5H-benzocyclohepta [5,6-c] pyridazine

A la solution de 8 g de chloro-3 dihydro-6,7 5H-benzocyclohepta [5,6-c] pyridazine (exemple 1—C) dans 70 ml d'acide sulfurique concentré, on ajoute lentement en 15 minutes environ la solution de 3,85 g de nitrate de potassium dans 50 ml d'acide sulfurique concentré.

On verse le mélange réactionnel dans 300 ml d'eau glacée et essore le précipité formé.

On le rince abondamment avec de l'eau puis on le sèche.

On chromatographie sur colonne de silice. En éluant avec le mélange hexane-acétate d'éthyle 3/1 (vol/vol) on obtient le produit attendu.

F: 174°C; Poids 5,4 g; Rendement 57%.

En poursuivant l'élution, on obtient une petite quantité d'un dérivé mononitré identifié comme le composé nitro-8 correspondant.

B) SR 95777 A

On chauffe au reflux pendant 17 heures, le mélange de 3,88 g du dérivé nitré obtenu ci-dessus et 5,08 g de diéthylamino-2 éthylamine dans 150 ml de n-butanol.

On évapore le n-butanol sous vide et chromatographie le résidu sur colonne de silice.

Par élution avec le mélange acétate d'éthyle-hexane-ammoniaque (89/10/1) vol/vol et après élimination d'impuretés en tête, on obtient le produit attendu.

Poids 2,57 g; Rendement 50%.

Dichlorhydrate

On dissout la base obtenue ci-dessus dans l'isopropanol chaud et ajoute un excès d'acide chlorhydrique concentré. On laisse cristalliser le dichlorhydrate puis on l'essore rince avec de l'éther isopropylique et recristallise dans un mélange isopropanol-éther éthylique.

On obtient 2,6 g de dichlorhydrate.

F: 198—200°C.

Exemple 22

Dichlorhydrate de (morpholino-2 éthylamino)-3 nitro-8 dihydro-6,7 5H benzocyclohepta [5,6-c] pyridazine (SR 96 100 A)

(I) $X = (CH_2)_3$ ; $R_1 = 8\text{-}NO_2$ ; $R_2 = H$ ; Y = NH

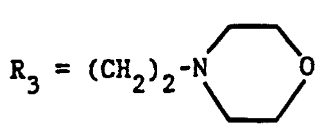

On opère comme dans l'exemple 21B) à partir du dérivé chloré nitré en 8 obtenu à l'exemple 21A) et de morpholino-2 éthylamine.

De la même façon, on obtient le produit attendu isolé comme dichlorhydrate.

F=206—207°C.

Exemples 23 et 24

En opérant comme dans l'exemple 21 mais en faisant varier le dérivé aminé utilisé dans l'étape B), on obtient les composés (I) du tableau 3.

TABLEAU 3

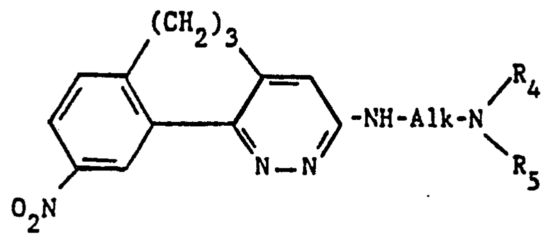

| N° Exemple | N° Code SR | Alk | $-N<^{R_4}_{R_5}$ | Base ou sel F : °C |
|---|---|---|---|---|
| 23 | 44681 A | $(CH_2)_2$ | $-N<^{CH_3}_{CH_3}$ | Dichlorhydrate 0,5 $H_2O$ 227-229°C |
| 24 | 44697 A | $(CH_2)_2$ | $-N<^{CH-(CH_3)_2}_{CH-(CH_3)_2}$ | Base 124-127°C |

Exemple 25

Dichlorhydrate d'amino-10 (diéthylamino-2 éthylamino)-3 dihydro-6,7 5H-benzocyclohepta (5,6-c] pyridazine (SR 95776 A)

(I) $X=(CH_2)_3$; $R_1=10\text{-}NH_2$; $R_2=H$

$$Y=NH;\ R_3=(CH_2)_2-N<^{C_2H_5}_{C_2H_5}$$

A la solution de 1,3 g du composé nitré de l'exemple 21 dans 50 ml de méthanol, on ajoute 0.1 g de palladium sur charbon à 5% et hydrogène à température et pression ordinaires pendant 3 heures.

On filtre le catalyseur et évapore le solvant. On recristallise le résidu dans un mélange isopropanol-éther anhydre.

On obtient le produit attendu.

F: 182°C; Poids 0,77 g Rendement 60%.

Exemple 26

Chlorhydrate de[(oxo-2 morpholino)-2 éthylamino]-3 dihydro-6,7 5H-benzocyclohepta [5,6-c] pyridazine (SR 95696 A)

(I) $X = (CH_2)_3$ ; $R_1 = R_2 = H$ ; $Y = NH$

$R_3 = (CH_2)_2$- 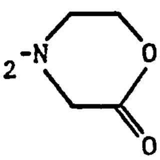

A la solution de 2 g de[(hydroxy-2 éthylamino)-2 éthylamino]-3 dihydro-6,7 5H benzocyclohepta [5,6-c] pyridazine (exemple 5) dans 35 ml de méthanol, on ajoute une solution de méthylate de sodium préparée à partir de 0,23 g de sodium et 25 ml de méthanol. Après une heure d'agitation à température ambiante, on ajoute 0,8 ml de chloracétate d'éthyle et poursuit l'agitation à température ambiante, sous atmosphère d'argon, pendant 15 heures.

On évapore le méthanol et reprend le résidu dans une solution aqueuse saturée de chlorure de sodium.

On extrait avec de l'acétate d'éthyle, sèche la solution organique et évapore à siccité sous vide.

Le résidu huileux est purifié par chromatographie sur colonne de silice. En éluant avec le mélange acétate d'éthyle-méthanol 8—2 vol/vol on obtient 1,7 g du produit attendu.

Rendement 74%.

Chlorhydrate

On dissout la base ci-dessus dans le méthanol et fait barbotter un courant d'acide chlorhydrique gazeux. On évapore à siccité et redissout le résidu dans le minimum de méthanol. Par addition d'éther anhydre, on précipite le chlorhydrate. On l'essore et recristallise dans le mélange éthanol à 95-éther.

F: 211°C.

Analyse pour $C_{19}H_{22}N_4O_2$, HCl (374,86)

| | | | |
|---|---|---|---|
| Calculé | C: 60,87 | H: 6,18 | N: 14,95 |
| Trouvé | 60,71 | 6,20 | 15,03 |

Exemple 27

Dichlorhydrate de (diéthylamino-3 propylamino)-3 dihydro-5,6 benzo [h] cinnoline (SR 95746 A)

(i) $X=(CH_2)_2$; $R_1=R_2=H$; $Y=NH$

$R_3=(CH_2)_3$— 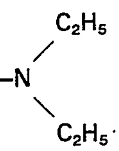

A) (oxo-1 tétrahydro-1,2,3,4 naphtyl-2) glycolate d'éthyle.

On opère comme dans l'exemple 1—A en utilisant comme cétone de départ l'alpha tétralone.

Par chromatographie sur colonne de silice, en éluant avec le mélange hexane-acétate d'éthyle 60—40 vol/vol on obtient le produit attendu sous forme d'huile.

Rendement 63%.

B) Tétrahydro-2,3,5,6, benzo [h] cinnolinone-3.

A partir du produit obtenu ci-dessus, on opére comme dans l'exemple 1—B.

Par refroidissement du mélange réactionnel à 0°C le produit attendu cristallise. On l'essore et lave avec de l'éther isopropylique.

F: 236°C.

C) Chloro-3 dihydro-5,6 benzo [h] cinnoline.

A partir du produit préparé ci-dessus et en opérant comme dans l'exemple 1—C on obtient le produit chloré.

F: 154°C; Rendement 60%.

D) SR 95746 A.

On chauffe au reflux pendant 24 heures le mélange de 2,16 g du dérivé chloré obtenu ci-dessus et 3,9 g de diéthylamino-3 propylamine dans 100 ml de n-butanol. On évapore le solvant à siccité sous vide et reprend le résidu dans l'acétate d'éthyle. On extrait avec une solution aqueuse diluée d'acide chlorhydrique et sépare la phase aqueuse qu'on alcalinise par du carbonate de potassium. On extrait avec de l'acétate d'éthyle et lave la solution avec de l'eau. On sèche la solution sur sulfate de sodium puis évapore le solvent à siccité sous vide. On purifie le résidu par chromatographie sur colonne de silice.

En éluant avec le mélange acétate d'éthyle-méthanol-ammoniaque 85—10—5 (vol/vol) on obtient une huile.

Poids: 0,55 g.

Dichlorhydrate

On dissout 0,5 g de base dans l'isopropanol et ajoute 0,27 ml d'acide chlorhydrique concentré.

Le chlorhydrate cristallise. On l'essore et on le lave avec de l'éther. On recristallise 2 fois dans l'isopropanol pour obtenir le dichlorhydrate.

F: 140°C; Rendement 65%.

Analyse avec 1,25 $H_2O$

| | C: | H: | N: |
|---|---|---|---|
| Calculé | 56,21 | 7,57 | 13,80 |
| Trouvé | 56,26 | 8,08 | 13,70 |

Exemple 28

Dichlorhydrate de (morpholino-2 éthylamino)-3 dihydro-5,6 benzo [h] cinnoline (SR 95695 A)

(I) $X = (CH_2)_2$ ; $R_1 = R_2 = H$ ; $Y = NH$

$R_3 = (CH_2)_2$- 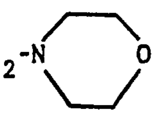

On opère comme dans l'exemple 27D) en remplaçant la diéthylamino-3 propylamine par une quantité équivalente de morpholino-2 éthylamine.

De la même façon, on obtient le produit attendu sous forme du dichlorhydrate.

F: 224°C (éthanol à 95).

Le sel cristallise avec 2 molécules d'eau.

Analyse $C_{18} H_{22} N_4O$, 2HCl, $2H_2O$ (419,33)

| | C: | H: | N: |
|---|---|---|---|
| Calculé | 51,55 | 6,72 | 13,36 |
| Trouvé | 51,66 | 6,55 | 13,10 |

Exemple 29

Dichlorhydrate de (diéthylamino-2 éthylamino)-3 dihydro-5,6 benzo [h] cinnoline (SR 96054)

(I) $X=(CH_2)_2$; $R_1=R_2=H$; Y=NH

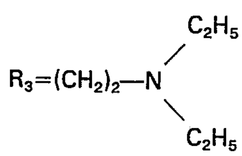

On opère comme dans l'exemple 27 et obtient de la même façon le produit attendu isolé comme dichlorhydrate.

F: 177—178°C.

Exemple 30

Dichlorhydrate de (morpholino-2 éthylamino)-3 benzo [h] cinnoline (SR 95679 A)

(I) $X = -CH = CH-$ ; $R_1 = R_2 = H$ ; $Y = NH$

$R_3 = (CH_2)_2$- 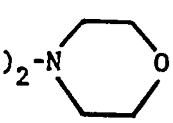

On chauffe à 135°C sous argon pendant 6 heures le mélange de 1,25 g du dérivé chloré obtenu à l'exemple 27C), 3 g de morpholino-2 éthylamine at 0,3 g de chlorure d'ammonium.

On verse le mélange réactionnel dans l'eau et extrait avec de l'acétate d'éthyle. On extrait la phase organique avec de l'acide sulfurique 2N et sépare la phase aqueuse. On alcalinise celle-ci avec du carbonate de sodium et extrait avec de l'acétate d'éthyle. On sèche la solution.

Celle-ci content 2 constituants qu'on sépare par chromatographie sur colonne de silice en éluant avec le mélange acétate d'éthyle-méthanol 95—5 vol/vol.

On obtient d'abord le produit du titre: poids 0,400 g puis en poursuivant l'élution on isole 0,300 g du produit dihydrogéné correspondant identique en tout point au produit décrit à l'exemple 28.

Dichlorhydrate

On dissout les 0,400 g de base dans l'isopropanol chaud et ajoute 0,22 ml d'acide chlorhydrique concentré. Le chlorhydrate précipite par refroidissement. On l'essore et recristallise dans l'éthanol à 95. F: 210°C (décomposition) solide jaune.

Analyse avec 1,5 molécule d'eau

$C_{18}H_{20}N_4O$, 2HCl, $1{,}5H_2O$ (408,22)

| | | | |
|---|---|---|---|
| Calculé | C: 52,94 | H: 6,17 | N: 13,72 |
| Trouvé | 52,95 | 6,32 | 13,51 |

Exemple 31

Dichlorhydrate de (diéthylamino-2 éthylamino)-3 benzo [h] cinnoline (SR 96055 A)

(I) X=CH=CH; $R_1$=$R_2$=H; Y=NH

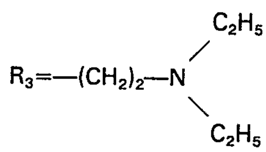

On opère comme dans l'exemple 30 en remplaçant la morpholino-2 éthylamine par une quantité équivalente de diéthylamino-2 éthylamine. De la même façon, on isole le produit attendu.

Base F: 90°C.

Dichlorhydrate F: 137—138°C (cristallisé avec une molécule d'eau).

Exemple 32

Dichlorhydrate de méthyl-5 (morpholine-2 éthylamino)-3 benzo [h] cinnoline (SR 96057 A)

$CH_3$

(I) X = -CH = C- ; $R_1$ = $R_2$ = H ; Y = NH

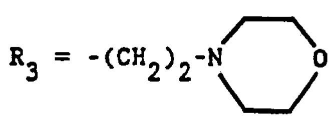

A) Méthyl-5 tétrahydro-2,3,5,6 benzo [h] cinnolinone-3.

On opère comme dans l'exemple 27, paragraphes A et B à partir de méthyl-3 dihydro-3,4 2H naphtalènone-1.

F: 218°C.

B) Chloro-3 méthyl-5 benzo [h] cinnoline.

On chauffe à 80°C pendant 4 heures, 7,6 g de la cinnolinone préparée ci-dessus et 50 ml d'oxychlorure de phosphore.

On verse le mélange sur de la glace pilée et extrait avec de l'acétate d'éthyle. On lave la solution organique avec de l'eau, sèche sur sulfate de sodium et évapore le solvant à siccité.

On purifie par chromatographie sur colonne de silice.

En éluant avec le mélange acétate d'éthyle-hexane (50/50, vol/vol) on obtient le produit sous forme de cristaux jaune pâle.

C) SR 96057 A.

On opère comme dans l'exemple 1D) à partir du dérivé chloré obtenu ci-dessus et de morpholino-2 éthylamine.

De la même façon, on obtient le produit attendu isolé sous forme de dichlorhydrate.

F: 176—177°C.

Exemple 33

Dichlorhydrate de chloro-10 (diéthylamino-2 éthylamino)-3 dihydro-6,7 5H benzocyclohepta [5,6-c] pyridazine (SR 95878 A)

(I) X=$(CH_2)_3$; $R_1$=10-Cl; $R_2$=H; Y=NH

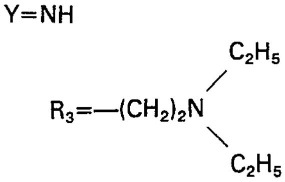

A) Amino-10 chloro-3 dihydro-6,7 5H benzocyclohepta [5,6-c] pyridazine.

Au mélange de 2,75 g de chloro-3 nitro-10 dihydro-6,7 5H benzocyclohepta [5,6-c] pyridazine (exemple 21A) et 2 g de poudre de fer dans 100 ml d'éthanol, on ajoute 8 ml d'acide acétique et porte au reflux pendant 4 heures. On laisse en suite le mélange réactionnel pendant 15 heures à 20°C et filtre sur Celite. On évapore l'alcool et reprend le résidu par l'eau et le chlorure de méthylène.

On sépare la phase aqueuse et on l'alcalinise par la soude. On extrait 2 fois avec du chlorure de méthylène et sèche la solution organique sur sulfate de sodium. On évapore le solvant sous vide.

On obtient un solide jaune (1,6 g). p. F: 171—172°C.

B) Dichloro-3,10 dihydro-6,7 5H benzocyclohepta [5,6-c] pyridazine.

On dissout 4,5 g du dérivé aminé obtenu ci-dessus dans 22 ml d'eau et 5 ml d'acide chlorhydrique concentré. On refroidit la solution à 0°C et ajoute rapidement une solution aqueuse acide de nitrite de sodium (1,4 g) en maintenant la température du mélange inférieure à 7°C.

On laisse sous agitation pendant 15 minutes et ajoute rapidement le mélange réactionnel à une solution de 2,25 g de chlorure cuivreux dans 30 ml d'eau acidifiée par l'acide chlorhydrique.

On laisse le mélange sous agitation pendant 2 heures à température ambiante et essore le précipité formé qu'on lave avec de l'eau. On le redissout dans le chlorure de méthylène et lave avec de l'eau. On sèche la solution organique sur sulfate de sodium et évapore le solvant à siccité.

On obtient 4,3 g du produit attendu.

F: 115°C.

C) SR 95878 A.

On opère comme dans l'exemple 1D) à partir du dérivé chloré obtenu ci-dessus.

De la même façon on obtient le produit attendu isolé sous forme de dichlorhydrate.

F: 188°C (cristallisé avec une molécule d'eau).

Exemple 34

Chlorhydrate de (diméthylamino-2 éthoxy)-3 dihydro-5,6 benzo [h] cinnoline (SR 95745 A)

(I) $X=(CH_2)_2$; $R_1=R_2=H$; $Y=O$

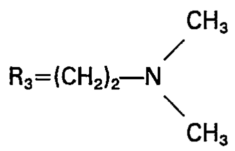

On chauffe à 80°C sous atmosphère d'azote le mélange de 5,84 g de diméthylamino-2 éthanol et 0,5 g d'hydrure de sodium. Après refroidissement à température ambiante, on ajoute 2,16 g du dérivé chloré obtenu à l'exemple 27C) et chauffe à nouveau à 80°C pendant 1 heure 30. Après refroidissement, on dilue le mélange réactionnel avec de l'acétate d'éthyle, on lave la solution organique avec de l'eau puis on extrait avec une solution aqueuse diluée d'acide chlorhydrique. On sépare la phase aqueuse, on alcalinise avec du carbonate de potassium et on extrait avec de l'acétate d'éthyle. On lave la phase organique avec de l'eau, sèche sur sulfate de sodium et évapore le solvant à siccité.

On purifie le résidu par chromatographie sur colonne de silice. En éluant avec le mélange acétate d'éthyle-méthanol-ammoniaque 85—10—5 vol/vol, on obtient une huile orangé (2 g).

Rendement 67%.

Chlorhydrate

On dissout 2 g de base dans l'isopropanol et ajoute 0,57 ml d'acide chlorhydrique concentré. Le chlorhydrate précipite par addition d'éther. On l'essore et recristallise dans le mélange isopropanol-éther.

On obtient 1,45 g du produit attendu.

F: 179°C; Rendement 65%.

Analyse $C_{18}H_{23}N_3O$, NCl (333,84)

| | | | |
|---|---|---|---|
| Calculé | C: 64,75 | H: 7,24 | N: 12,58 |
| Trouvé | 64,39 | 7,40 | 12,45 |

Exemple 35

Chlorhydrate de (diméthylamino-2 éthoxy)-3 dihydro-6,7 5H-benzocyclohepta [5,6-c] pyridazine (SR 95 822 A)

On opère comme dans l'exemple 34 à partir du dérivé chloré de l'exemple 1C). De la même façon on isole le chlorhydrate attendu; F: 182°C.

Analyse pour C19 H25 N3 O, HCl (347,87)

| | | | |
|---|---|---|---|
| Calculé | C: 65,59 | H: 7,53 | N: 12,07 |
| Trouvé | 65,72 | 7,81 | 11,96 |

Exemple 36

Chlorhydrate de (diéthylamino-2 éthylthio)-3 dihydro-5,6 benzo [h] cinnoline (SR 95 743 A)

(I) $X=(CH_2)_2$; $R_1=R_2=H$; Y=S

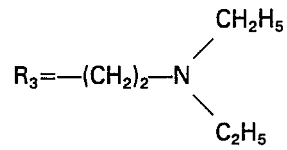

A) Tétrahydro-2,3,5,6 benzo [h] cinnolinethione-3.

On dissout 5,34 g de tétrahydro-2,3,5,6 benzo [h] cinnolinone-3 (exemple 27B) dans 27 ml d'acétonitrile et 27 ml d'éther diméthylique de l'éthylèneglycol. On ajoute ensuite 16,2 g de pentasulfure de phosphore puis à température ambiante, on ajoute par petites fractions sous bonne agitation 4,2 g de bicarbonate de sodium.

On chauffe ensuite pendant 4 heures à 90°C puis on évapore les solvants sous vide puis verse le résidu dans l'eau. On essore les cristaux et sèche.

On obtient 5 g du produit attendu.

F: 236—240°C; Rendement 85%.

B) SR 95743 A.

On dissout 3 g de la thione ci-dessus dans une solution d'éthylate de sodium dans l'éthanol obtenue à partir de 0,7 g de sodium. On ajoute sous agitation 2,58 g de chlorhydrate de diéthylamino-2 chloro-1 éthane et laisse le mélange pendant 15 heures à température ambiante. On évapore le solvant à siccité sous vide et reprend le résidu dans l'acétate d'éthyle. On lave la solution organique avec de l'eau, puis on l'extrait avec de l'acide chlorhydrique dilué. On sépare la phase aqueuse et on l'alcalinise avec du carbonate de potassium. On extrait avec de l'acétate d'éthyle, lave avec de l'eau et sèche sur sulfate de sodium. On évapore le solvant à siccité. On obtient une huile brune (2,45 g) qu'on purifie par chromatographie sur colonne de silice. En éluant avec le mélange acétate d'éthyle-méthanol 90—10, vol/vol, après élimination d'impuretés en tête, on obtient le produit attendu (1,6 g).

Chlorhydrate

A la solution de 0,5 g de base dans l'isopropanol on ajoute 0,14 ml d'acide chlorhydrique concentré puis on précipite le chlorhydrate par addition d'éther. On essore les cristaux et recristallise dans l'éthanol absolu.

On obtient 0,4 g du produit attendu; F: 228°C.

Analyse C18 H23 N3 S, HCl (349,90)

| | C: | H: | N: |
|---|---|---|---|
| Calculé | 61,17 | 6,91 | 12,00 |
| Trouvé | 61,36 | 6,89 | 11,88 |

Exemple 37

Chlorhydrate de (diéthylamino-2 éthylthio)-3 dihydro-6,7 5H-benzocyclohepta [5,6-c] pyridazine (SR 95820 A)

(I) $X=(CH_2)_3$; $R_1=R_2=H$; Y=S

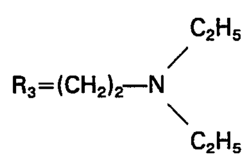

On opère comme dans l'exemple 36 à partir de la pyridazinone de l'exemple 1—B. De la même façon on obtient le produit attendu; F: 174°C.

Analyse C19 H25 N3 S, HCl (363,93)

| | C: | H: | N: |
|---|---|---|---|
| Calculé | 62,70 | 7,20 | 11,54 |
| Trouvé | 62,53 | 7,11 | 11,50 |

Les produits selon l'invention ont été étudiés en ce qui concerne leur action thérapeutique. Notamment l'intéraction des produits selon l'invention vis à vis des récepteurs muscariniques cholinergiques a été déterminée.

Chez les mammifères, il existe deux sous classes de récepteurs cholinergiques muscariniques: les récepteurs $M_1$ et $M_2$.

Les récepteurs de type $M_1$ sont concentrés dans certaines zones de cerveau telles que l'hippocampe, le cortex cérébral, le striatum ainsi que dans les ganglions sympathiques. Ces sites de liaison peuvent être

sélectivement marqués par la [3H] pirenzépine ([3H]PZ). Ceux de type $M_2$ prédominent dans le coeur et dans l'iléon et peuvent être marqués par le [3H] N-méthylscopolamine ([3H]NMS). Afin de déterminer la sélectivité de nos molécules, vis à vis des sites $M_1$ et $M_2$, nous avons étudié leur intéraction in vitro avec les fixations à haute affinité de la [3H] PZ et de la [3H] NMS sur des membranes d'hippocampe de rat et de muscle lisse d'iléon de cobaye, respectivement.

Methodologies

A) Recherche d'une affinité pour le récepteur cholinergique muscarinique de type $M_1$.

L'intéraction des molécules avec les récepteurs muscariniques de type $M_1$ a été étudiée en mesurant in vitro sur un homogénat d'hippocampe de rat, le déplacement de la pirenzépine tritiée ([3H]PZ) de ses sites de fixation spécifiques. Des aliquots (10 µl) d'un homogénat d'hippocampe de rat à 5% (P/v) dans un tampon $Na_2HPO_4$ (50 mM, pH 7,40) sont incubés 2 h à 4°C en présence de [3H]PZ (76 Ci/nmole; 1 nM final) et de concentrations croissantes en produits à étudier. Le volume final est de 2 ml. La réaction est arrêtée par centrifugation 10 mn à 50.000 xg. Après décantation et lavage des culots, la radioactivité fixée est comptée par scintillation liquide. La fixation non spécifique est déterminée en présence de 10 µM de sulfate d'atropine. La concentration inhibitrice 50 ($Cl_{50}$) est déterminée graphiquement.

(Réf.: Watson J.D., Roeskoe W.R. and Yamamura H.I., Live Sci., 31, 2019—2029, 1982).

B) Recherche d'une affinité pour le récepteur cholinergique muscarinique de type $M_2$.

L'intéraction avec les récepteurs muscariniques de type $M_2$ a été étudiée en mesurant in vitro sur un homogénat de muscle lisse d'iléon de cobaye, le déplacement de la N-méthyl-scopolamine tritiée ([3H]NMS) de ses sites de fixation spécifiques. Des aliquots (50 µl) d'un homogénat de muscle lisse d'iléon de cobaye à 0,625% (P/v) dans du tampon MEPES (20 mM) contenant NaCl (100 mM) et Mg $Cl_2$ (10 mM) (pH final: 7,5), sont incubés 20 mn à 30°C en présence de [3H]NMS (85 Ci/nmole; 0,3 nM final) et de concentrationscroissantes en produits à tester. Le volume final est de 1 ml. La réaction est arrêtée par centrifugation 5 mn à 15.000 xg. La fixation non spécifique est déterminée en présence de 10 µM de sulfate d'atropine.

(Réf.: Hammer R., Berrie C.P., Birdsall N.I.M., Burgen A.S.V. and Hulme E.C., Nature, 283, 90—92, 1980 Hulme E.C., Birdsall, N.I.M., Burgen A.S.V. and Mettha P., Mol. Pharmacol., 14, 737—750, 1978).

Resultats

Le tableau 4 indique les afinités des produits de l'invention pour les récepteurs $M_1$ et $M_2$. Les résultats sont exprimés en concentrations inhibitrices 50 pour cent (Cl 50), soit la concentration (en µM) qui induit un déplacement de 50% du ligand tritié fixé aux récepteurs membranaires. La $Cl_{50}$ de déplacement de la $^3$H-pirenzépine représente l'affinité pour le récepteur $M_1$; la CI50 de déplacement de la $^3$H—NMS représente l'affinité pour le récepteur $M_2$.

De plus dans la 3e colonne on a indiqué le rapport r entre les $Cl_{50}$ $M_1$ et $M_2$ qui exprime la sélectivité des produits vis à vis de l'un des types de récepteurs.

TABLEAU 4

| Produit N° | $^3$H-Pirenzepine ($M_1$) CI 50 µ M | $^3$H-NMS ($M_2$) CI 50 µ M | r = ($M_2$/$M_1$) |
|---|---|---|---|
| SR 95695 A | 2,6 | 100 | 38 |
| SR 95679 A | 3,1 | 100 | 32 |
| SR 95746 A | 1,5 | 25 | 17 |
| SR 95743 A | 0,7 | 10 | 14 |
| SR 95745 A | 3,9 | 80 | 21 |
| SR 95639 A | 0,4 | 55 | 137 |
| SR 95640 A | 0,05 | 2 | 40 |
| SR 95776 A | 0,2 | 2 | 10 |
| SR 95777 A | 0,1 | 5,6 | 56 |

TABLEAU 4 suite

| Produit N° | $^{3}H$-Pirenzepine ($M_1$) CI 50 µ M | $^{3}H$-NMS ($M_2$) CI 50 µ M | r = ($M_2/M_1$)' |
|---|---|---|---|
| SR 95818 A | 0,24 | 12 | 50 |
| SR 95820 A | 0,07 | 2 | 29 |
| SR 95822 A | 1 | 9,5 | 9,5 |
| SR 95824 A | 0,2 | 25 | 125 |
| SR 44694 A | 0,15 | 3,6 | 24 |
| SR 44695 A | 0,45 | 4,5 | 10 |
| SR 44664 A | 0,18 | 2 | 11 |
| SR 44696 A | 0,7 | 6,5 | 10 |
| SR 44705 A | 0,18 | 3 | 16 |
| SR 44706 A | 3,6 | 30 | 8 |
| SR 44703 A | 0,1 | 4 | 40 |
| SR 44681 A | 0,26 | 9 | 35 |
| SR 44697 A | 0,3 | 3 | 10 |
| SR 95776 A | 0,2 | 2 | 10 |
| SR 95746 A | 1,5 | 25 | 17 |
| SR 95695 A | 2,6 | > 100 | > 38 |
| SR 95679 A | 3,1 | > 100 | > 32 |
| SR 95745 A | 3,9 | 80 | 21 |
| SR 95878 A | 0,04 | 1,1 | 26 |
| SR 95879 A | 0,14 | 1 | 7 |
| SR 96093 A | 1,2 | 50 | 42 |
| SR 95883 A | 0,13 | 4,5 | 35 |
| SR 96100 A | 0,4 | 44 | 110 |
| SR 96054 A | 0,21 | 7,5 | 36 |
| SR 96055 A | 0,32 | 17 | 53 |
| SR 96057 A | 3 | 43 | 14 |
| SR 44663 A | 0,16 | 5 | 37 |
| SR 96108 A | 1 | 40 | 40 |

Ces résultats montrent que les composés selon l'invention présentent une forte affinité pour les récepteurs cholinergiques muscariniques avec une spécificité marquée pour les récepteurs centraux de type $M_1$.

Par ailleurs certains des composés selon l'invention ont été soumis à une étude pharmacologique in vivo.

Etude Pharmacologique in vivo

La pirenzépine (PZ) est un antagoniste spécifique des récepteurs cholinergiques muscariniques centraux $M_1$. L'injection intrastriatale de PZ chez la souris induit un comportement rotatoire. On a étudié l'antagonisme de ce comportement par les produits selon l'invention.

Les produits selon l'invention sont injectés par voie intrapéritonéale (i.p.) après avoir été solubilisés dans l'eau distillée ou mis en suspension dans une solution de gomme arabique à 5%. Les contrôles sont réalisés par injection du solvant pur dans les mêmes conditions.

Les animaux utilisé sont des souris femelles (Swiss, CD 1, Charles River, France) de poids corporel compris entre 25 et 30 grammes.

La pirenzépine est mise en solution dans un tampon phosphate, le pH de la solution est 6.

Les produits à étudier ou leurs solvants sont injectés par voie i.p. sous un volume de 0,4 ml pour 20 g de poids corporel, 15 minutes avant une injection directe de pirenzépine à la dose de 1 µg de produit dans 1 µl de solvant dans le striatum droit de la souris, selon la méthode décrite par P. WORMS et al. dans Eur. J.Pharmaco. 1986, *121*, 395—401.

Le nombre de rotations contralatérales (sens opposé au côté de l'injection) a été compté pendant trois périodes de 2 minutes après injection de pirenzépine: minutes 2 à 4, 8 à 10 et 13 à 15. Chaque traitement comporte 3 à 4 doses et 10 animaux par dose. Pour chaque traitement, on calcule le nombre total de rotations et la pourcentage d-antagonism vis-à-vis du lot contrôle.

Pour chaque produit on détermine graphiquement la dose efficace 50 ($DE_{50}$): dose qui diminue de 50% le nombre de rotations in duites par la pirenzépine. Les résultats sont reportés dans le tableau 5.

TABLEAU 5

| Produits n° | Antagonisme Pirenzépine $DE_{50}$ mg/kg i.p. |
|---|---|
| SR 95695 A | 5 |
| SR 95679 A | 10 |
| SR 95746 A | 10-30 |
| SR 95743 A | 8 |
| SR 95745 A | 8 |
| SR 95639 A | 5 |
| SR 95640 A | 3 |
| SR 95696 A | 3 |
| SR 95777 A | 0,5 |
| SR 95817 A | 2 |
| SR 95818 A | 7 |
| SR 95819 A | 10 |
| SR 95820 A | 8 |
| SR 95822 A | 3 |
| SR 95824 A | 3 |
| SR 44650 A | 3 |
| SR 44664 A | 3 |
| SR 44681 A | 1 |
| SR 96093 A | 15 |
| SR 96054 A | 3 |
| SR 96055 A | 3 |
| SR 44663 A | 1 |
| SR 95878 A | 2 |
| SR 95879 A | 0,3 |
| SR 95883 A | 3 |

Par ailleurs des essais effectués sur quelques uns des composés selon l'invention ont mis en évidence que les composés (I) franchissent les barrières hématoencéphalique et digestive.

Enfin les composés selon l'invention n'ont manifesté aucun signe de toxicité apparente aux doses où ils sont actifs.

Par suite les composés (I) peuvent être utilisés en tant que médicaments et notamment dans les cas où se manifeste un déficit cholinergique cortical et en particulier dans le cas démences de type Alzheimer.

En conséquence selon un autre de ses aspects la présente demande concerne les compositions pharmaceutiques contenant au moins un des composés de formule (I) ou un de leurs sels en tant qu'ingrédient actif.

Dans les compositions pharmaceutiques de la présente invention pour l'administration orale, sublinguale, sous-cutanée, intramusculaire, intraveineuse, transdermique ou rectale, les ingrédients actifs de formule (I) ci-dessus peuvent administrés sous formes unitaires d'administration, en mélange avec des supports pharmaceutiques classiques, aux être humains notamment pour le traitement des démences séniles. Les formes unitaires d'administration appropriées comprennent les formes par voie orale telles

que les comprimés, les gélules, les poudres, les granulés et les solutions ou suspensions orales, les formes d'administration sublingale et buccale, les formes d'administration sous-cutanée, intramusculaire ou intraveineuse et les formes d'administration rectale.

Afin d'obtenir l'effet désiré, la dose de principe actif peut varier entre 50 et 2000 mg par jour.

Chaque dose unitaire peut contenir de 10 à 500 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicle pharmaceutique tel que la gélatine, l'amidon, le lactose, le stéarate de magnésium, le talc, le gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granulés dispersibles aans l'eau peuvent contenir l'ingrédient actif en mélange avec des agents de dispersion ou des agents mouillants, ou des agents de mise in suspension, comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Ainsi à titre d'exemple, on peut préparer des gélules à base d'un des composés des exemples 1 à 37 ayant la composition suivante:

| | |
|---|---|
| Principe actif | 25 mg |
| Lactose | 110 mg |
| Stéarate de magnésium | 5 mg |

en mélangeant intimement les ingrédients ci-dessus et en versant le mélange dans des gélules de gélatine dure.

**Revendications pour les Etats contractants: BE CH DE FR GB IT LI LU NL SE**

1. Composés tricycliques de formule générale:

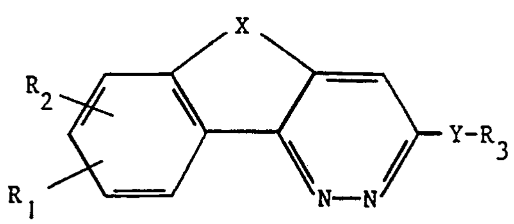

(I)

dans laquelle:

X représente un groupe $(CH_2)_n$ avec n représentant un entier égal à 2, 3 ou 4 ou encore un groupe vinylène ou un groupe méthylvinylène;

$R_1$ et $R_2$ considérés indépendamment représentent l'hydrogène ou un substituant occupant l'une des positions libres du cycle benzénique et choisi parmi les halogènes, un groupe alkyle inférieur, un groupe alcoxy inférieur, un groupe hydroxy, un groupe thiol, un groupe nitro, un groupe amino;

Y représente l'oxygène, le soufre ou un groupe —NH—;

$R_3$ represente:

un groupe

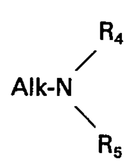

dans lequel Alk représente un groupe alkylène droit ou ramifié ayant de 2 à 5 atomes de carbone, $R_4$ et $R_5$ représentent chacun indépendamment l'hydrogène, un groupe alkyle inférieur, un groupe hydroxyalkyle inférieur ou encore $R_4$ et $R_5$ constituent avec l'atome d'azote auquel ils sont liés un groupe choisi parmi les

groupes pyrrolidinyl-1, pipéridino, morpholino, pipérazinyl-1, oxo-2 morpholino;

un groupe

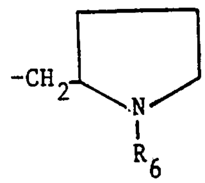

où $R_6$ représente un groupe alkyle inférieur ayant 1 à 4 atomes de carbone;

un groupe

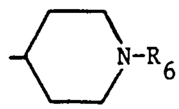

où $R_6$ est comme défini ci-dessus;

ainsi que leurs sels avec les acides minéraux ou organiques pharmaceutiquement acceptables.

2. Composés selon la revendication 1 où X représente un groupe $(CH_2)_n$ où n est égal à 2, 3 ou 4.

3. Composés selon la revendication 1 où X représente un groupe vinylène ou méthylvinylène.

4. Procédé de préparation des composés de formule (I) où X représente un groupe $(CH_2)_n$, n = 2, 3 ou 4, caractérisé en ce que:

on traite à chaud entre 60 et 150°C une benzocycloalkanone-1 par le glyoxylate d'éthyle ou l'acide glyoxylique pour former l'hydroxyester ou l'hydroxyacide:

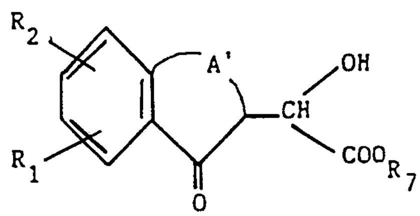

(a)

où A' représente $(CH_2)_n$ et $R_7$ est H ou $C_2H_5$

on chauffe cet hydroxyester avec de l'hydrate d'hydrazine au sein d'un solvant convenable pour former la pyridazone

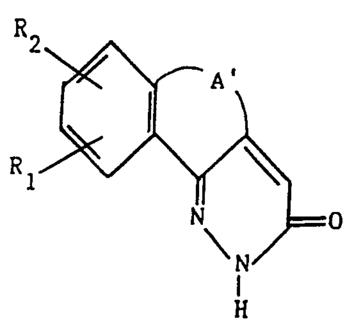

(b)

ou A' est tel que défini ci-dessus

où traite au reflux la pyridazone pa un excès d'oxychlorure ou d'oxybromure de phosphore pour former le dérivé halogéné correspondant

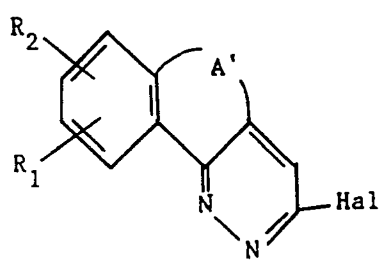

(c)

Hal = Cl, Br

on substitue le dérivé halogéné par chauffage avec un dérivé B-$R_3$ où B représente OH, SH ou $NH_2$ éventuellement en présence d'un activateur de la réaction pour former le composé (I) correspondant;

éventuellement on salifie le composé (I) selon un procédé connu.

5. Procédé de préparation des composés de formule (I), où X est $(CH_2)_n$ et $R_1$ en position para de X est autre que l'hydrogène alors que $R_2$ = H, caractérisé en ce que:

on nitre le dérivé chloré (c) où $R_1$ et $R_2$ représentent l'hydrogène pour obtenir le composé où $R_1$ est $NO_2$;

on substitue le dérivé chloré par chauffage avec un dérivé B-$R_3$ comme indiqué à la revendication 4;

éventuellement on transforme le dérivé nitré en dérivé aminé correspondant par réduction catalytique;

éventuellement on transforme le groupe amino en d'autres substituants par des réactions connues en elles-mêmes;

éventuellement on salifie le composé (I) ainsi obtenu.

6. Procédé de préparation des composés de formule (I) où X est $(CH_2)_n$ et $R_1$ en position para de X est autre que l'hydrogène alors que $R_2$ = H, caractérisé en ce que:

on nitre le dérivé chloré (c) où $R_1$ et $R_2$ représentent l'hydrogène pour obtenir le composé où $R_1$ est $NO_2$;

on transforme ce dérivé chloré (c) en dérivé aminé correspondant par réduction catalytique;

éventuellement on transforme le groupe amino en d'autres substituants par des réactions connues en elles-mêmes;

on substitue le dérivé chloré par chauffage avec un dérivé B-$R_3$ comme indiqué à la revendication 4;

éventuellement on salifie le composé (I) ainsi obtenu.

7. Procédé de préparation des composés de formule (I) où X est $(CH_2)_n$ et où Y représente un atome de soufre caractérisé en ce que:

on transforme la pyridazone (b) en thione correspondante par chauffage avec un excès de pentasulfure de phosphore en présence de bicarbonate de sodium au sein d'un solvant convenable;

on substitue la thione par action d'un halogénure Hal-$R_3$ où Hal représente un halogène choisi parmi le chlore ou le brome au sein d'un alcool et en présence de l'alcoolate de sodium correspondant ou au sein du diméthylformamide;

éventuellement on salifie le composé (I) ainsi obtenu.

8. Procédé de préparation des composés de formule (I) où X représente un groupe vinylène caractérisé en ce que:

on chauffe à 120—150°C le dérivé chloré (c) où A′ représente —$(CH_2)_2$— avec un excès du dérive $BR_3$ et en présence de chlorure d'ammonium;

on sépare le composé (I) où X représente un groupe vinylène du composé (I) où X représente un groupe éthylène qui s'est formé conjointement;

éventuellement on salifie le composé (I) ainsi isolé.

9. Procédé de préparation des composés de formule (I) dans laquelle X est le groupe méthylvinylène, $R_1$, $R_2$, Y et $R_3$ sont tels que définis dans la revendication 1, caractérisé en ce qu'il consiste:

à traiter la pyridazone de formule (b) dans laquelle A′ représente un groupe méthyléthylène par de l'oxychlorure de phosphore pour obtenir le composé de formule (c) dans laquelle A′ représente un groupe méthylvinylène;

à substituer le dérivé chloré obtenu par chauffage avec un dérivé B-$R_3$ où B représente OH, SH ou $NH_2$; éventuellement en présence d'un activateur de la réaction pour former le composé (I) correspondant;

éventuellement à salifier le composé (I) selon un procédé connu.

10. Procédé de préparation des composés de formule (I) dans laquelle

$R_3$ représente un groupe

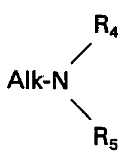

dans lequel $NR_4R_5$ désigne n groupe oxo-2 morpholino caractérisé en ce qu'il consiste à traiter les composés de formule (I) correspondants dans lesquels $NR_4R_5$ représente un groupe NH—$CH_2$—$CH_2$—OH par substitution de l'azote par le chloracétate d'éthyle et cyclisation de l'hydroxyester ainsi obtenu.

11. Compositions pharmaceutiques contenant à titre de principe actif, un composé de formule (I) en combinaison avec un véhicule pharmaceutiquement acceptable.

**Revendications pour les Etats contractants: AT ES**

1. Procédé pour l'obtention des composés tricycliques de formule générale:

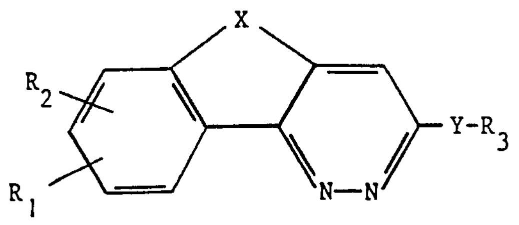

(I)

dans laquelle:

X représente un groupe $(CH_2)_n$ avec n représentant un entier égal à 2, 3 ou 4;

$R_1$ et $R_2$ considérés indépendamment représentent l'hydrogène ou un substituant occupant l'une des positions libres du cycle benzénique et choisi parmi les halogènes, un groupe alkyle inférieur, un groupe alcoxy inférieur, un groupe hydroxy, un groupe thiol, un groupe nitro, un groupe amino;

Y représente l'oxygène, le soufre ou un groupe —NH—;

$R_3$ représente:

un groupe

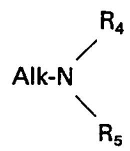

dans lequel Alk représente un groupe alkylène droit ou ramifié ayant de 2 à 5 atomes de carbone, $R_4$ et $R_5$ représentent chacun indépendamment l'hydrogène, un groupe alkyle inférieur, un groupe hydroxyalkyle inférieur ou encore $R_4$ et $R_5$ constituent avec l'atome d'azote auquel ils sont liés un groupe choisi parmi les groupes pyrrolidinyl-1, pipéridino, morpholino, pipérazinyl-1, oxo-2 morpholino;

un groupe

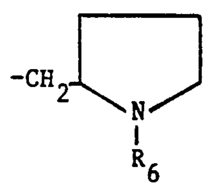

où $R_6$ représente un groupe alkyle inférieur ayant 1 à 4 atomes de carbone;

un groupe

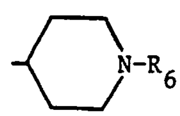

ou $R_6$ est comme défini ci-dessus;

ainsi que leurs sels avec les acides minéraux ou organiques pharmaceutiquement acceptables, caractérisé en ce que:

on traite à chaud entre 60 et 150°C une benzocycloalkanone-1 par le glyoxylate d'éthyle ou l'acide glyoxylique pour former l'hydroxyester ou l'hydroxyacide:

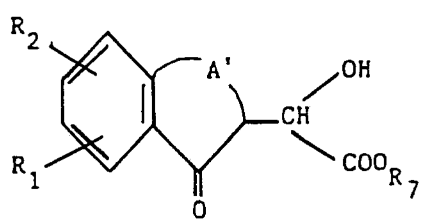

(a)

où A' représente $(CH_2)_n$ et $R_7$ est H ou $C_2H_5$

on chauffe cet hydroxyester avec de l'hydrate d'hydrazine au sein d'un solvant convenable pour former la pyridazone

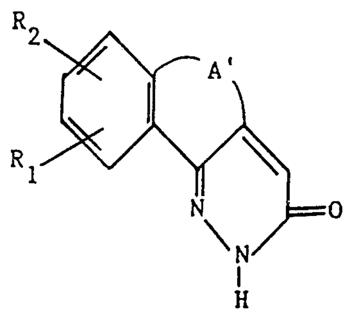

(b)

où A' est tel que défini ci-dessus

où traite au reflux la pyridazone par un excès d'oxychlorure ou d'oxybromure de phosphore pour former le dérivé halogéné correspondant.

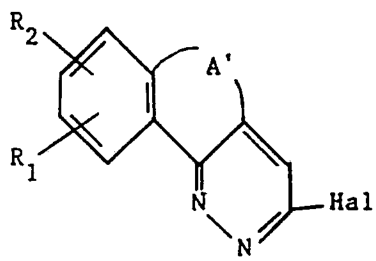

(c)

Hal = Cl, Br

on substitue le dérivé halogéné par chauffage avec un dérivé B-$R_3$ où B représente OH, SH ou $NH_2$ éventuellement en présence d'un activateur de la réaction pour former le composé (I) correspondant,

éventuellement on salifie le composé (I) selon un procédé connu.

2. Procédé selon la revendication 1, pour l'obtention des composés de formule (I), dans laquelle X est $(CH_2)_n$ et $R_1$ en position para de X est autre que l'hydrogène alors que $R_2$ = H, caractérisé en ce que:

on nitre le dérivé chloré (c) où $R_1$ et $R_2$ représentent l'hydrogène pour obtenir le composé où $R_1$ est $NO_2$;

on substitue le dérivé chloré par chauffage avec un dérivé B-$R_3$ comme inidiqué à la revendication 1;

éventuellement on transforme le dérivé nitré en dérivé aminé correspondant par réduction catalytique;

éventuellement on transforme le groupe amino en d'autres substituants par des réactions connues en elles-mêmes;

éventuellement on salifie le composé (I) ainsi obtenu.

3. Procédé selon la revendication 1 pour l'obtention des composés de formule (I) dans laquelle X est $(CH_2)_n$ et $R_1$ en position para de X est autre que l'hydrogène alors que $R_2$ = H, caractérisé en ce que:

on nitre le dérivé chloré (c) où $R_1$ et $R_2$ représentent l'hydrogène pour obtenir le composé où $R_1$ est $NO_2$;

on transforme ce dérivé chloré (c) en dérivé aminé correspondant par réduction catalytique;

éventuellement on transforme le groupe amino en d'autres substituants par des réactions connues en elles-mêmes;

on substitue le dérivé chloré par chauffage avec un dérivé B-$R_3$ comme indiqué à la revendication 1;

éventuellement on salifie le composé (I) ainsi obtenu.

4. Procédé selon la revendication 1, pour l'obtention des composés de formule (I) où X est $(CH_2)_n$ et ou Y représente un atome de soufre caractérisé en ce que:

on transforme la pyridazone (b) en thione correspondante par chauffage avec un excès de pentasulfure de phosphore en présence de bicarbonate de sodium au sein d'un solvant convenable;

on substitue la thione par action d'un halogénure Hal-$R_3$ où Hal représente un halogène choisi parmi le chlore ou le brome au sein d'un alcool et en présence de l'alcoolate de sodium correspondant ou au sein du diméthylformamide;

éventuellement on salifie le composé (I) ainsi obtenu.

5. Procédé pour l'obtention des composés tricycliques de formule générale:

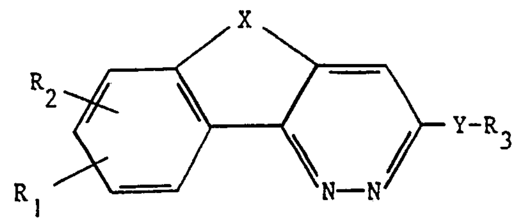

(I)

dans laquelle:

X représente un groupe vinylène;

$R_1$ et $R_2$ considérés indépendamment représentent l'hydrogène ou un substituant occupant l'une des positions libres du cycle benzénique et choisi parmi les halogènes, un groupe alkyle inférieur, un groupe alcoxy inférieur, un groupe hydroxy, un groupe thiol, un groupe nitro, un groupe amino;

Y représente l'oxygène, le soufre ou un groupe —NH—;

$R_3$ represente:

un groupe

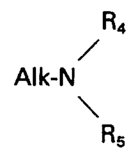

dans lequel Alk représente un groupe alkylène droit ou ramifié ayant de 2 à 5 atomes de carbone, $R_4$ et $R_5$ représentent chacun indépendamment l'hydrogène, un groupe alkyle inférieur, un groupe hydroxyalkyle inférieur ou encore $R_4$ et $R_5$ constituent avec l'atome d'azote auquel ils sont liés un groupe choisi parmi les groupes pyrrolidinyl-1, pipéridino, morpholino, pipérazinyl-1, oxo-2 morpholino;

un groupe

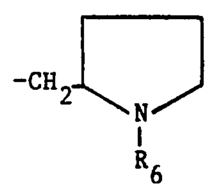

où $R_6$ représente un groupe alkyle inférieur ayant 1 à 4 atomes de carbone;

un groupe

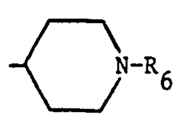

ou $R_6$ est comme défini ci-dessus;

ainsi que leurs sels avec les acides minéraux ou organiques pharmaceutiquemente acceptables, caractérisé en ce que:

on chauffe à 120—150°C le dérivé chloré (c) où A' représente $—(CH_2)_2—$ avec un excès du dérivé $BR_3$, tel que défini à la revendication 1 et en présence de chlorure d'ammonium;

on sépare le composé (I) où X représente un groupe vinylène du composé (I) où X représente un groupe éthylène qui s'est formé conjointement;

éventuellement on salifie le composé (I) ainsi isolé.

6. Procédé pour l'obtention des composés tricycliques de formule générale:

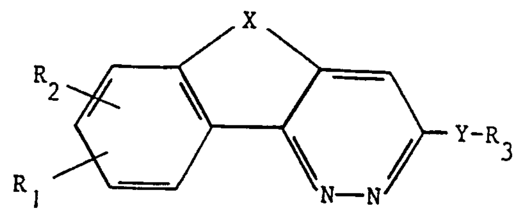

(I)

dans laquelle:

X est le groupe méthylvinylène, $R_1$, $R_2$, Y et $R_3$ sont tels que définis dans la revendication 1, caractérisé en ce qu'il consiste:

à traiter la pyridazone de formule (b) dans laquelle A' représente un groupe méthyléthylène par de l'oxychlorure de phosphore pour obtenir le composé de formule (c) dans laquelle A' représente un groupe méthylvinylène;

à substituer le dérivé chloré obtenu par chauffage avec un dérivé B-$R_3$ où B représente OH, SH ou $NH_2$; éventuellement en présence d'un activateur de la réaction pour former le composé (I) correspondant;

éventuellement à salifier le composé (I) selon un procédé connu.

7. Procédé selon la revendication 1 pour l'obtention des composés tricycliques de formule (I) dans laquelle

$R_3$ représente un groupe

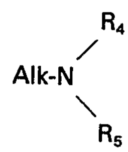

dans lequel $NR_4R_5$ désigne n groupe oxo-2 morpholino caractérisé en ce qu'il consiste à traiter les composés de formule (I) correspondants dans lesquels $NR_4R_5$ représente un groupe $NH—CH_2—CH_2—OH$ par substitution de l'azote par le chloracétate d'éthyle et cyclisation de l'hydroxyester ainsi obtenu.

8. Utilisation des composés tricycliques de formule:

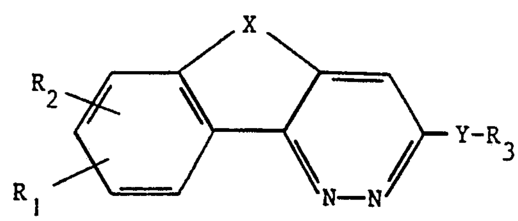

(I)

dans laquelle:

X représente un groupe $(CH_2)_n$ avec n représentant un entier égal à 2, 3 ou 4, ou encore un groupe vinylène ou un grupue méthyl vinylène,

$R_1$ et $R_2$ considérés indépendamment représentent l'hydrogène ou un substituant occupant l'une des positions libres du cycle benzénique et choisi parmi les halogènes, un groupe alkyle inférieur, un groupe alcoxy inférieur, un groupe hydroxy, un groupe thiol, un groupe nitro, un groupe amino;

Y représente l'oxygène, le soufre ou un groupe —NH—;

$R_3$ represente:

un groupe

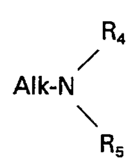

dans lequel Alk représente un groupe alkylène droit ou ramifié ayant de 2 à 5 atomes de carbone, $R_4$ et $R_5$ représentent chacun indépendamment l'hydrogène, un groupe alkyle inférieur, un groupe hydroxyalkyle inférieur ou encore $R_4$ et $R_5$ constituent avec l'atome d'azote auquel ils sont liés un groupe choisi parmi les groupes pyrrolidinyl-1, pipéridino, morpholino, pipérazinyl-1, oxo-2 morpholino;

un groupe

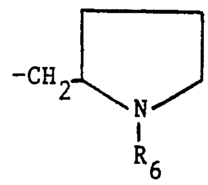

où $R_6$ représente un groupe alkyle inférieur ayant 1 à 4 atomes de carbone;

un groupe

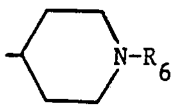

où $R_6$ est comme défini ci-dessus;

ainsi que leurs sels avec les acides minéraux ou organiques pharmaceutiquement acceptables, pour la préparation de compositions pharmaceutiques, notamment agonistes des récepteurs cholinergiques.

**Revendications pour l'Etat contractant: GR**

1. Composés tricycliques de formule générale:

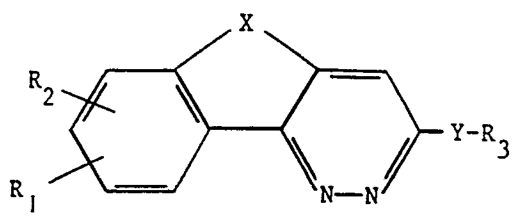

(I)

dans laquelle:

X représente un groupe $(CH_2)_n$ avec n représentant un entier égal à 2, 3 ou 4 ou encore un groupe vinylène ou un groupe méthylvinylène;

$R_1$ et $R_2$ considérés indépendamment représentent l'hydrogène ou un substituant occupant l'une des positions libres du cycle benzénique et choisi parmi les halogènes, un groupe alkyle inférieur, un groupe alcoxy inférieur, un groupe hydroxy, un groupe thiol, un groupe nitro, un groupe amino;

Y représente l'oxygène, le soufre ou un groupe —NH—;

$R_3$ represente:

un groupe

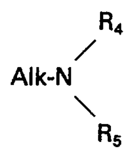

dans lequel Alk représente un groupe alkylène droit ou ramifié ayant de 2 à 5 atomes de carbone, $R_4$ et $R_5$ représentent chacun indépendamment l'hydrogène, un groupe alkyle inférieur, un groupe hydroxyalkyle inférieur ou encore $R_4$ et $R_5$ constituent avec l'atome d'azote auquel ils sont liés un groupe choisi parmi les groupes pyrrolidinyl-1, pipéridino, morpholino, pipérazinyl-1, oxo-2 morpholino;

un groupe

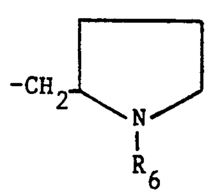

où $R_6$ représente un groupe alkyle inférieur ayant 1 à 4 atomes de carbone;

un groupe

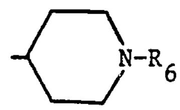

où $R_6$ est comme défini ci-dessus;
ainsi que leurs sels avec les acides minéraux ou organiques pharmaceutiquement acceptables.

2. Composés selon la revendication 1 où X représente un groupe $(CH_2)_n$ où n est égal à 2, 3 ou 4.

3. Composés selon la revendication 1 où X représente un groupe vinylène ou méthylvinylène.

4. Procédé de préparation des composés de formule (I) où X représente un groupe $(CH_2)_n$, n = 2, 3 ou 4, caractérisé en ce que:

on traite à chaud entre 60 et 150°C une benzocycloalkanone-1 par le glyoxylate d'éthyle ou l'acide glyoxylique pour former l'hydroxyester ou l'hydroxyacide:

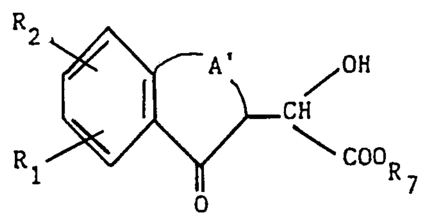

(a)

où A' représente $(CH_2)_n$ et $R_7$ est H ou $C_2H_5$

on chauffe cet hydroxyester avec de l'hydrate d'hydrazine au sein d'un solvant convenable pour former la pyridazone

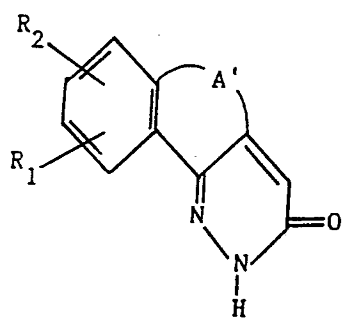

(b)

où A' est tel que défini ci-dessus

on traite au reflux la pyridazone pa un excès d'oxychlorure ou d'oxybromure de phosphore pour former le dérivé halogéné correspondant

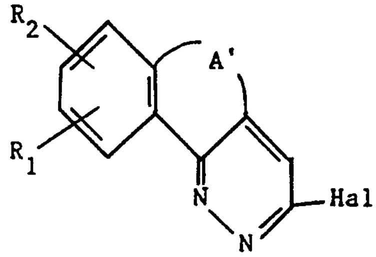

(c)

Hal = Cl, Br

on substitue le dérivé halogéné par chauffage avec un dérivé B-$R_3$ où B représente OH, SH ou $NH_2$ éventuellement en présence d'un activateur de la réaction pour former le composé (I) correspondant;

éventuellement on salifie le composé (I) selon un procédé connu.

5. Procédé de préparation des composés de formule (I), où X est $(CH_2)_n$ et $R_1$ en position para de X est autre que l'hydrogène alors que $R_2$ = H, caractérisé en ce que:

on nitre le dérivé chloré (c) où $R_1$ et $R_2$ représentent l'hydrogène pour obtenir le composé où $R_1$ est $NO_2$;

on substitue le dérivé chloré par chauffage avec un dérivé B-$R_3$ comme indiqué à la revendication 4;

éventuellement on transforme le dérivé nitré en dérivé aminé correspondant par réduction catalytique;

éventuellement on transforme le groupe amino en d'autres substituants par des réactions connues en elles-mêmes;

éventuellement on salifie le composé (I) ainsi obtenu.

6. Procédé de préparation des composés de formule (I) où X est $(CH_2)_n$ et $R_1$ en position para de X est autre que l'hydrogène alors que $R_2 = H$, caractérisé en ce que:

on nitre le dérivé chloré (c) où $R_1$ et $R_2$ représentent l'hydrogène pour obtenir le composé où $R_1$ est $NO_2$;

on transforme ce dérivé chloré (c) en dérivé aminé correspondant par réduction catalytique;

éventuellement on transforme le groupe amino en d'autres substituants par des réactions connues en elles-mêmes;

on substitue le dérivé chloré par chauffage avec un dérivé B-$R_3$ comme indiqué à la revendication 4;

éventuellement on salifie le composé (I) ainsi obtenu.

7. Procédé de préparation des composés de formule (I), où X est $(CH_2)_n$ et où Y représente un atome de soufre caractérisé en ce que:

on transforme la pyridazone (b) en thione correspondante par chauffage avec un excès de pentasulfure de phosphore en présence de bicarbonate de sodium au sein d'un solvant convenable;

on substitue la thione par action d'un halogénure Hal-$R_3$ où Hal représente un halogène choisi parmi le chlore ou le brome au sein d'un alcool et en présence de l'alcoolate de sodium correspondant ou au sein du diméthylformamide;

éventuellement on salifie le composé (I) ainsi obtenu.

8. Procédé de préparation des composés de formule (I) où X représente un groupe vinylène caractérisé en ce que:

on chauffe à 120—150°C le dérivé chloré (c) où A' représente $—(CH_2)_2—$ avec un excès du dérivé $BR_3$ et en présence de chlorure d'ammonium;

on sépare le composé (I) où X représente un groupe vinylène du composé (I) où X représente un groupe éthylène qui s'est formé conjointement;

éventuellement on salifie le composé (I) ainsi isolé.

9. Procédé de préparation des composés de formule (I) dans laquelle X est le groupe méthylvinylène, $R_1$, $R_2$, Y et $R_3$ sont tels que définis dans la revendication 1, caractérisé en ce qu'il consiste:

à traiter la pyridazone de formule (b) dans laquelle A' représente un groupe méthyléthylène par de l'oxychlorure de phosphore pour obtenir le composé de formule (c) dans laquelle A' représente un groupe méthylvinylène;

à substituer le dérivé chloré obtenu par chauffage avec un dérivé B-$R_3$ où B représente OH, SH ou $NH_2$; éventuellement en présence d'un activateur de la réaction pour former le composé (I) correspondant;

éventuellement à salifier le composé (I) selon un procédé connu.

10. Procédé de préparation des composés de formule (I) dans laquelle

$R_3$ représente un groupe

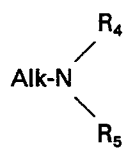

dans lequel $NR_4R_5$ désigne un groupe oxo-2 morpholino caractérisé en ce qu'il consiste à traiter les composés de formule (I) correspondants dans lesquels $NR_4R_5$ représente un groupe $NH—CH_2—CH_2—OH$ par substitution de l'azote par le chloracétate d'éthyle et cyclisation de l'hydroxyester ainsi obtenu.

11. Procédé pour la préparation de compositions pharmaceutiques, caractérisé en ce qu'il consiste à inclure une quantité efficace d'un composé selon l'une quelconque des revendications 1 à 3 dans un excipient convenable.

**Patentansprüche für die Vertragsstaaten: BE CH DE FR GB IT LI LU NL SE**

1. Tricyclische Verbindungen der allgemeinen Formel:

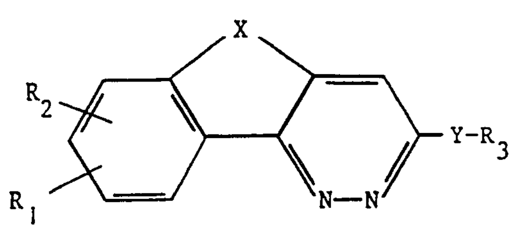

(I)

worin:

X für eine Gruppe $(CH_2)_n$, in der n eine ganze Zahl gleich 2, 3 oder 4 ist, oder auch eine Vinylengruppe

oder eine Methylvinylengruppe steht;

$R_1$ und $R_2$, getrennt betrachtet, Wasserstoff oder einen Substituenten darstellen, der eine der freien Positionen des Benzolrings einnimmt und ausgewählt ist aus Halogenen, einer Niedrigalkylgruppe, einer Niedrigalkoxygruppe, einer Hydroxygruppe, einer Thiolgruppe, einer Nitrogruppe, einer Aminogruppe;

Y Sauerstoff, Schwefel oder eine Gruppe —NH— bedeutet;

$R_3$ darstellt:

eine Gruppe

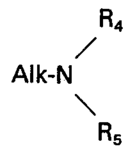 ,

worin Alk für eine gerade oder verzweige Alkylengruppe mit 2 bis 5 Kohlenstoffatomen steht, $R_4$ und $R_5$ jeweils unabhängig Wasserstoff, eine Niedrigalkylgruppe, eine Niedrighydroxyalkylgruppe darstellen, oder $R_4$ und $R_5$ mit dem Stickstoffatom, an das sie gebunden sind, eine Gruppe, ausgewählt aus den Gruppen 1-Pyrrolidinyl, Piperidino, Morpholino, 1-Piperazinyl, 2-Oxo-morpholino, bilden;

eine Gruppe

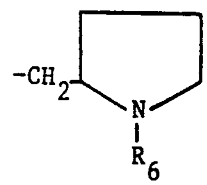

worin $R_6$ eine Niedrigalkylgruppe mit 1 bis 4 Kohlenstoffatomen darstellt;

eine Gruppe

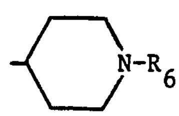

worin $R_6$ wie oben definiert ist;

sowie die Salze derselben mit pharmazeutisch akzeptablen Mineral- oder organischen Säuren.

2. Verbindungen nach Anspruch 1, worin X eine Gruppe $(CH_2)_n$ darstellt, in der n gleich 2, 3 oder 4 ist.

3. Verbindungen nach Anspruch 1, worin X eine Vinylen- oder Methylvinylengruppe darstellt.

4. Verfahren zur Herstellung von Verbindungen der Formel (I), worin X eine Gruppe $(CH_2)_n$ darstellt, n = 2, 3 oder 4, dadurch gekennzeichnet, daß;

man ein 1-Benzocycloalkanon mit Ethylglyoxylat oder Glyoxylsäure zwischen 60 und 150°C wärmebehandelt, um den Hydroxyester oder die Hydroxysäure

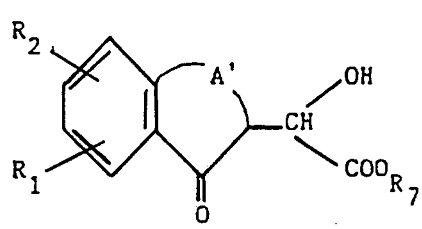

(a)

worin A' für $(CH_2)_n$ steht und $R_7$ H oder $C_2H_5$ ist zu bilden,

man diesen Hydroxyester mit Hydrazinhydrat in einem geeigneten Lösungsmittel erhitzt, um das Pyridazon

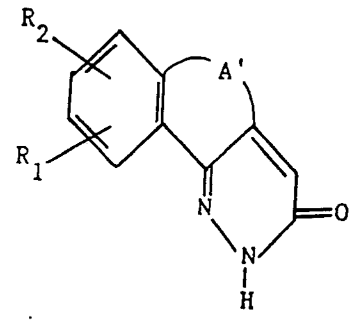

(b)

worin A' wie oben definiert ist zu bilden.

man das Pyridazon mit einem Überschuß an Phosphoroxychlorid oder -oxybromid unter Rückfluß behandelt, um das entsprechende Halogenderivat

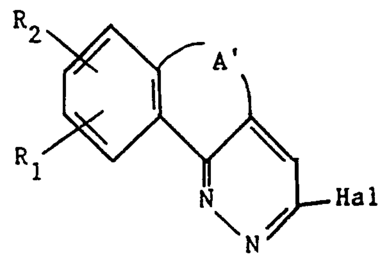

(c)

Hal = Cl, Br zu bilden,

man das Halogenderivat durch Erhitzen mit einem B-$R_3$- Derivat, worin B für OH, SH oder $NH_2$ steht, gegebenenfalls in Gegenwart eines Reaktionsaktivators substituiert, um die entsprechende Verbindung (I) zu bilden;

man gegebenenfalls die Verbindung (I) nach einem bekannten Verfahren in das Salz überführt.

5. Verfahren zur Herstellung von Verbindungen der Formel (I), worin X für $(CH_2)_n$ steht und $R_1$ in der para-Position von X nicht Wasserstoff ist, während $R_2$ = H, dadurch gekennzeichnet, daß

man das Chlorderivat (c), worin $R_1$ und $R_2$ Wasserstoff darstellen, nitriert, um die Verbindung, in der $R_1$ für $NO_2$ steht, zu erhalten;

man das Chlorderivat durch Erhitzen mit einem B-$R_3$-Derivat, wie in Anspruch 4 ausgeführt, substituiert;

man gegebenenfalls das Nitroderivat durch katalytische Reduktion in das entsprechende Aminoderivat überführt;

man gegebenenfalls die Aminogruppe mittels an sich bekannter Verfahren in andere Substituenten überführt;

man gegebenenfalls die so erhaltene Verbindung (I) in das Salz überführt.

6. Verfahren zur Herstellung von Verbindungen der Formel (I), worin X für $(CH_2)_n$ steht und $R_1$ in der para-Position von X nicht Wasserstoff ist, während $R_2$ = H, dadurch gekennzeichnet, daß

man das Chlorderivat (c), worin $R_1$ und $R_2$ Wasserstoff darstellen, nitriert, um die Verbindung, in der $R_1$ für $NO_2$ steht, zu erhalten;

man dieses Chlorderivat (c) durch katalytische Reduktion in das entsprechende Aminoderivat überführt;

man gegebenenfalls die Aminogruppe mittels an sich bekannter Reaktionen in andere Substituenten überführt;

man das Chlorderivat durch Erhitzen mit einem Derivat B-$R_3$, wie in Anspruch 4 ausgeführt, substituiert;

man gegebenenfalls die so erhaltene Verbindung (I) in das Salz überführt.

7. Verfahren zur Herstellung von Verbindungen der Formel (I), worin X für $(CH_2)_n$ steht und worin Y ein Schwefelatom darstellt, dadurch gekennzeichnet, daß:

man das Pyridazon (b) durch Erhitzen mit einem Überschuß an Phosphorpentasulfid in Gegenwart von Natriumbicarbonat in einem geeigneten Lösungsmittel in das entsprechende Thion überführt;

man das Thion durch Umsetzung eines Halogenids Hal-$R_3$, worin Hal ein Halogen, ausgewählt aus Chlor oder Brom, darstellt, in einem Alkohol und in Gegenwart des entsprechenden Natriumalkoholats oder in Dimethylformamid substituiert;

man gegebenenfalls die so erhaltene Verbindung (I) in das Salz überführt.

8. Verfahren zur Herstellung von Verbindungen der Formel (I), worin X eine Vinylengruppe darstellt, dadurch gekennzeichnet, daß:

man das Chlorderivat (c), worin A' für $—(CH_2)_2—$ steht, mit einem Überschuß des Derivats $BR_3$ in Gegenwart von Ammoniumchlorid auf 120—150°C erhitzt;

man die Verbindung (I), worin X eine Vinylengruppe darstellt, von der Verbindung (I), worin X eine Ethylengruppe darstellt und die sich gleichzeitig gebildet hat, abtrennt;

man gegebenenfalls die so isolierte Verbindung (I) in das Salz überführt.

9. Verfahren zur Herstellung von Verbindungen der Formel (I), worin X die Gruppe Methylvinylen ist, $R_1$, $R_2$, Y und $R_3$ wie in Anspruch 1 definiert sind, dadurch gekennzeichnet, daß es darin besteht, daß:

das Pyridazon der Formel (b), worin A' eine Methylethylengruppe darstellt, mit Phosphoroxychlorid behandelt wird, um die Verbindung der Formel (c) zu erhalten, in der A' eine Methylvinylengruppe darstellt;

das erhaltene Chlorderivat durch Erhitzen mit einem Derivat B-$R_3$, worin B für OH, SH oder $NH_2$ steht, substituiert wird, gegebenenfalls in Gegenwart eines Reaktionsaktivators, um die entsprechende Verbindung (I) zu bilden;

gegebenenfalls die Verbindung (I) nach einem bekannten Verfahren in das Salz übergeführt wird.

10. Verfahren zur Herstellung von Verbindungen der Formel (I), worin

$R_3$ eine Gruppe

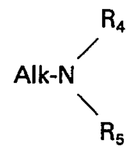

darstellt, in der $NR_4R_5$ eine 2-Oxo-morpholinogruppe bezeichnet, dadurch gekennzeichnet, daß es darin besteht, daß die entsprechenden Verbindungen der Formel (I), in denen $NR_4R_5$ eine Gruppe $NH—CH_2—CH_2—OH$ darstellt, durch Substitution des Stickstoffs mit Ethylchloracetat und Zyklisierung des so erhaltenen Hydroxyesters behandelt werden.

11. Pharmazeutische Zusammensetzungen, die als Wirkstoff eine Verbindung der Formel (I) in Kombination mit einem pharmazeutisch akzeptablen Vehikel enthalten.

**Patentansprüche für die Vertragsstaaten: AT ES**

1. Verfahren zur Herstellung von tricyclischen Verbindungen der allgemeinen Formel

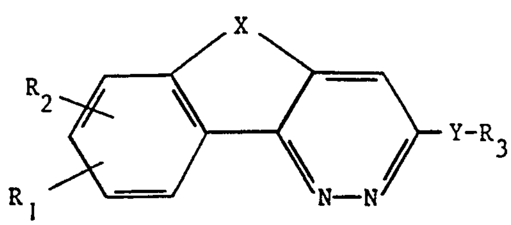

(I)

worin:

X für eine Gruppe $(CH_2)_n$, in der n eine ganze Zahl gleich 2, 3 oder 4 ist;

$R_1$ und $R_2$, getrennt betrachtet, Wasserstoff oder einen Substituenten darstellen, der eine der freien Positionen des Benzolrings einnimmt und ausgewählt ist aus Halogenen, einer Niedrigalkylgruppe, einer Niedrigalkoxygruppe, einer Hydroxygruppe, einer Thiolgruppe, einer Nitrogruppe, einer Aminogruppe;

Y Sauerstoff, Schwefel oder eine Gruppe —NH— bedeutet;

$R_3$ darstellt:

eine Gruppe

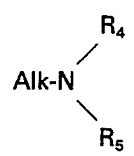

,

worin:

Alk für eine gerade oder verzweige Alkylengruppe mit 2 bis 5 Kohlenstoffatomen steht,

$R_4$ und $R_5$ jeweils unabhängig Wasserstoff, eine Niedrigalkylgruppe, eine Niedrighydroxyalkylgruppe darstellen, oder $R_4$ und $R_5$ mit dem Stickstoffatom, an das sie gebunden sind, eine Gruppe, ausgewählt aus den Gruppen 1-Pyrrolidinyl, Piperidino, Morpholino, 1-Piperazinyl, 2-Oxo-morpholino, bilden;

eine Gruppe

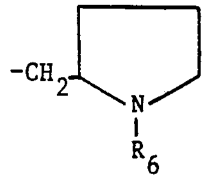

worin $R_6$ eine Niedrigalkylgruppe mit 1 bis 4 Kohlenstoffatomen darstellt;
eine Gruppe

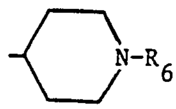

worin $R_6$ wie oben definiert ist;
sowie der Salze derselben mit pharmazeutisch akzeptablen Mineral- oder organischen Säuren, dadurch gekennzeichnet, daß

man ein 1-Benzocycloalkanon mit Ethylglyoxylat oder Glyoxylsäure zwischen 60 und 150°C wärmebehandelt, um den Hydroxyester oder die Hydroxysäure

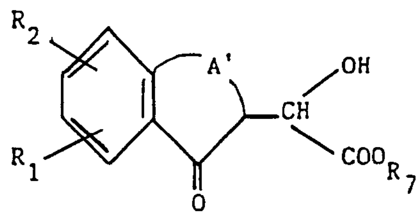

(a)

worin A' für $(CH_2)_n$ steht und $R_7$ H oder $C_2H_5$ ist zu bilden,

man diesen Hydroxyester mit Hydrazinhydrat in einem geeigneten Lösungsmittel erhitzt, um das Pyridazon

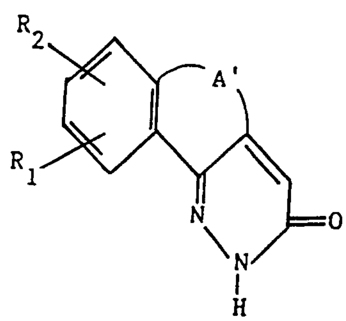

(b)

worin A' wie oben definiert ist zu bilden,

man das Pyridazon mit einem Überschuß an Phosphoroxychlorid oder -oxybromid unter Rückfluß behandelt, um das entsprechende Halogenderivat

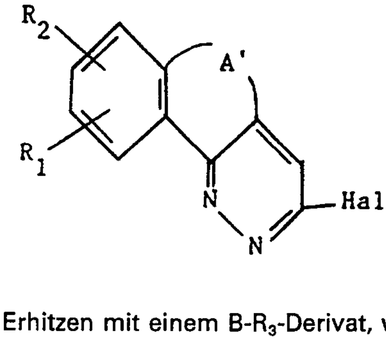

(c)

Hal = Cl, Br zu bilden,

man das Halogenderivat durch Erhitzen mit einem B-$R_3$-Derivat, worin B für OH, SH oder $NH_2$ steht,

gegebenenfalls in Gegenwart eines Reaktionsaktivators substituiert, um die entsprechende Verbindung (I) zu bilden;

man gegebenenfalls die Verbindung (I) nach einem bekannten Verfahren in das Salz überführt.

2. Verfahren nach Anspruch 1 zur Herstellung von Verbindungen der Formel (I), worin X für $(CH_2)_n$ steht und $R_1$ in der para-Position von X nicht Wasserstoff ist, während $R_2$ = H, dadurch gekennzeichnet, daß

man das Chlorderivat (c), worin $R_1$ und $R_2$ Wasserstoff darstellen, nitriert, um die Verbindung, in der $R_1$ für $NO_2$ steht, zu erhalten;

man das Chlorderivat durch Erhitzen mit einem B-$R_3$-Derivat, wie in Anspruch 4 ausgeführt, substituiert;

man gegebenenfalls das Nitroderivat durch katalytische Reduktion in das entsprechende Aminoderivat überführt;

man gegebenenfalls die Aminogruppe mittels an sich bekannter Verfahren in andere Substituenten überführt;

man gegebenenfalls die so erhaltene Verbindung (I) in das Salz überführt.

3. Verfahren nach Anspruch 1 zur Herstellung von Verbindungen der Formel (I), worin X für $(CH_2)_n$ steht und $R_1$ in der para-Position von X nicht Wasserstoff ist, während $R_2$ = H, dadurch gekennzeichnet,

man das Chlorderivat (c), worin $R_1$ und $R_2$ Wasserstoff darstellen, nitriert, um die Verbindung, in der $R_1$ für $NO_2$ steht, zu erhalten;

man dieses Chlorderivat (c) durch katalytische Reduktion in das entsprechende Aminoderivat überführt;

man gegebenenfalls die Aminogruppe mittels an sich bekannter Reaktionen in andere Substituenten überführt;

man das Chlorderivat durch Erhitzen mit einem Derivat B-$R_3$, wie in Anspruch 1 ausgeführt, substituiert;

man gegebenenfalls die so erhaltene Verbindung (I) in das Salz überführt.

4. Verfahren nach Anspruch 1 zur Herstellung von Verbindungen der Formel (I), worin X für $(CH_2)_n$ steht und worin Y ein Schwefelatom darstellt, dadurch gekennzeichnet, daß:

man das Pyridazon (b) durch Erhitzen mit einem Überschuß an Phosphorpentasulfid in Gegenwart von Natriumbicarbonat in einem geeigneten Lösungsmittel in das entsprechende Thion überführt;

man das Thion durch Umsetzung eines Halogenids Hal-$R_3$, worin Hal ein Halogen, ausgewählt aus Chlor oder Brom, darstellt, in einem Alkohol und in Gegenwart des entsprechenden Natriumalkoholats oder in Dimethylformamid substituiert;

man gegebenenfalls die so erhaltene Verbindung (I) in das Salz überführt.

5. Verfahren zur Herstellung von tricyclischen Verbindungen der allgemeinen Formel:

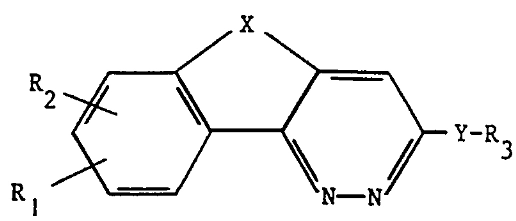

(I)

worin:

X eine Vinylengruppe darstellt,

$R_1$ und $R_2$, getrennt betrachtet, Wasserstoff oder einen Substituenten darstellen, der eine der freien Positionen des Benzolrings einnimmt und ausgewählt ist aus Halogenen, einer Niedrigalkylgruppe, einer Niedrigalkoxygruppe, einer Hydroxygruppe, einer Thiolgruppe, einer Nitrogruppe, einer Aminogruppe;

Y Sauerstoff, Schwefel oder eine Gruppe —NH— bedeutet;

$R_3$ darstellt:

eine Gruppe

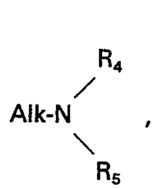

worin:

Alk für eine gerade oder verzweige Alkylengruppe mit 2 bis 5 Kohlenstoffatomen steht,

$R_4$ und $R_5$ jeweils unabhängig Wasserstoff, eine Niedrigalkylgruppe, eine Niedrighydroxyalkylgruppe darstellen, oder $R_4$ und $R_5$ mit dem Stickstoffatom, an das sie gebunden sind, eine Gruppe, ausgewählt aus den Gruppen 1-Pyrrolidinyl, Piperidino, Morpholino, 1-Piperazinyl, 2-Oxo-morpholino, bilden;

eine Gruppe

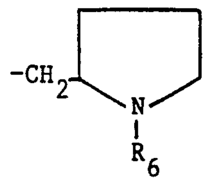

worin $R_6$ eine Niedrigalkylgruppe mit 1 bis 4 Kohlenstoffatomen darstellt;

eine Gruppe

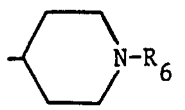

worin $R_6$ wie oben definiert ist;

sowie der Salze derselben mit pharmazeutisch akzeptablen Mineral- oder organischen Säuren, dadurch gekennzeichnet, daß

man das Chlorderivat (c), worin A' für $-(CH_2)_2-$ steht, mit einem Überschuß des Derivats $BR_3$ in Gegenwart von Ammoniumchlorid auf 120—150°C erhitzt;

man die Verbindung (I), worin X eine Vinylengruppe darstellt, von der Verbindung (I), worin X eine Ethylengruppe darstellt und die sich gleichzeitig gebildet hat, abtrennt;

man gegebenenfalls die so isolierte Verbindung (I) in das Salz überführt.

6. Verfahren zur Herstellung von tricyclischen Verbindungen der allgemeinen Formel

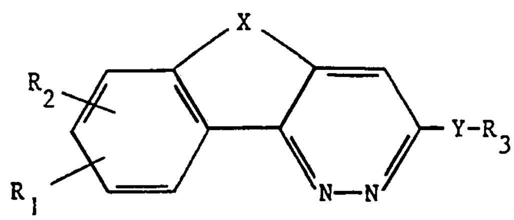

(I)

worin:

X die Gruppe Methylvinylen ist, $R_1$, $R_2$, Y und $R_3$ wie in Anspruch 1 definiert sind, dadurch gekennzeichnet, daß es darin besteht, daß:

das Pyridazon der Formel (b), worin A' eine Methylethylengruppe darstellt, mit Phosphoroxychlorid behandelt wird, um die Verbindung der Formel (c) zu erhalten, in der A' eine Methylvinylengruppe darstellt;

das erhaltene Chlorderivat durch Erhitzen mit einem Derivat $B-R_3$, worin B für OH, SH oder $NH_2$ steht, substituiert wird, gegebenenfalls in Gegenwart eines Reaktionsaktivators, um die entsprechende Verbindung (I) zu bilden;

gegebenenfalls die Verbindung (I) nach einem bekannten Verfahren in das Salz übergeführt wird.

7. Verfahren nach Anspruch 1 zur Herstellung von tricyclischen Verbindungen der Formel (I), worin:

$R_3$ eine Gruppe

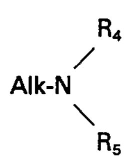 darstellt,

in der $NR_4R_5$ eine 2-Oxo-morpholinogruppe bezeichnet, dadurch gekennzeichnet, daß es darin besteht, daß die entsprechenden Verbindungen der Formel (I), in denen $NR_4R_5$ eine Gruppe $NH-CH_2-CH_2-OH$ darstellt, durch Substitution des Stickstoffs mit Ethylchloracetat und Zyklisierung des so erhaltenen Hydroxyesters behandelt werden.

8. Verwendung der tricyclischen Verbindungen der Formel:

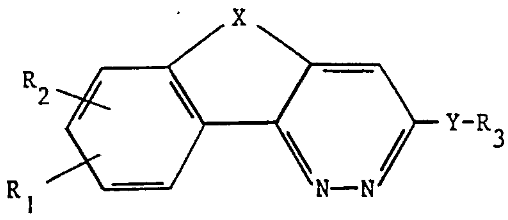

(I)

worin:

X für eine Gruppe $(CH_2)_n$, in der n eine ganze Zahl gleich 2, 3 oder 4 ist, oder auch eine Vinylengruppe oder eine Methylvinylengruppe steht;

$R_1$ und $R_2$, getrennt betrachtet, Wasserstoff oder einen Substituenten darstellen, der eine der freien Positionen des Benzolrings einnimmt und ausgewählt ist aus Halogenen, einer Niedrigalkylgruppe, einer Niedrigalkoxygruppe, einer Hydroxygruppe, einer Thiolgruppe, einer Nitrogruppe, einer Aminogruppe;

Y Sauerstoff, Schwefel oder eine Gruppe —NH— bedeutet;

$R_3$ darstellt:

eine Gruppe

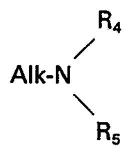

,

worin:

Alk für eine gerade oder verzweige Alkylengruppe mit 2 bis 5 Kohlenstoffatomen steht,

$R_4$ und $R_5$ jeweils unabhängig Wasserstoff, eine Niedrigalkylgruppe, eine Niedrighydroxyalkylgruppe darstellen, oder $R_4$ und $R_5$ mit dem Stickstoffatom, an das sie gebunden sind, eine Gruppe, ausgewählt aus den Gruppen 1-Pyrrolidinyl, Piperidino, Morpholino, 1-Piperazinyl, 2-Oxo-morpholino, bilden;

eine Gruppe

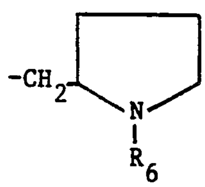

worin $R_6$ eine Niedrigalkylgruppe mit 1 bis 4 Kohlenstoffatomen darstellt;

eine Gruppe

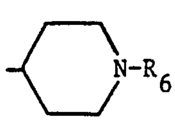

worin $R_6$ wie oben definiert ist;

sowie der Salze derselben mit pharmazeutisch akzeptablen Mineral- oder organischen Säuren zur Herstellung von pharmazeutischen Zusammensetzungen, insbesondere Agonisten für cholinerge Rezeptoren.

**Patentansprüche für den Vertragsstaat: GR**

1. Tricyclische Verbindungen der allgemeinen Formel:

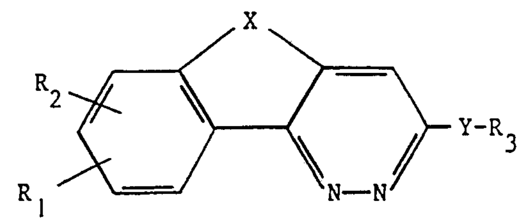

(I)

worin:

X für eine Gruppe $(CH_2)_n$, in der n eine ganze Zahl gleich 2, 3 oder 4 ist, oder auch eine Vinylengruppe oder eine Methylvinylengruppe steht;

$R_1$ und $R_2$, getrennt betrachtet, Wasserstoff oder einen Substituenten darstellen, der eine der freien Positionen des Benzolrings einnimmt und ausgewählt ist aus Halogenen, einer Niedrigalkylgruppe, einer Niedrigalkoxygruppe, einer Hydroxygruppe, einer Thiolgruppe, einer Nitrogruppe, einer Aminogruppe;

Y Sauerstoff, Schwefel oder eine Gruppe —NH— bedeutet;

$R_3$ darstellt:

eine Gruppe

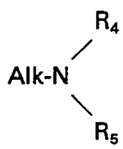

,

worin:

Alk für eine gerade oder verzweige Alkylengruppe mit 2 bis 5 Kohlenstoffatomen steht,

$R_4$ und $R_5$ jeweils unabhängig Wasserstoff, eine Niedrigalkylgruppe, eine Niedrighydroxyalkylgruppe darstellen, oder $R_4$ und $R_5$ mit dem Stickstoffatom, an das sie gebunden sind, eine Gruppe, ausgewählt aus den Gruppen 1-Pyrrolidinyl, Piperidino, Morpholino, 1-Piperazinyl, 2-Oxo-morpholino, bilden;

eine Gruppe

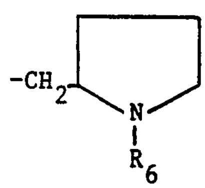

worin $R_6$ eine Niedrigalkylgruppe mit 1 bis 4 Kohlenstoffatomen darstellt;

eine Gruppe

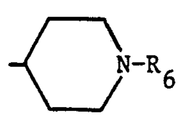

worin $R_6$ wie oben definiert ist;

sowie die Salze derselben mit pharmazeutisch akzeptablen Mineral- oder organischen Säuren.

2. Verbindungen nach Anspruch 1, worin X eine Gruppe $(CH_2)_n$ darstellt, in der n gleich 2, 3 oder 4 ist.

3. Verbindungen nach Anspruch 1, worin X eine Vinylen- oder Methylvinylengruppe darstellt.

4. Verfahren zur Herstellung von Verbindungen der Formel (I), worin X eine Gruppe $(CH_2)_n$ darstellt, n = 2, 3 oder 4, dadurch gekennzeichnet, daß:

man ein 1-Benzocycloalkanon mit Ethylglyoxylat oder Glyoxylsäure zwischen 60 und 150°C wärmebehandelt, um den Hydroxyester oder die Hydroxysäure

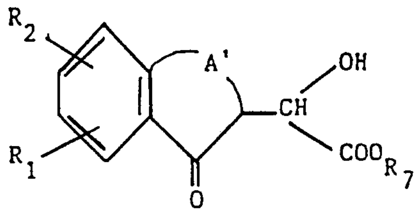

(a)

worin A' für $(CH_2)_n$ steht und $R_7$ H oder $C_2H_5$ ist zu bilden,

man diesen Hydroxyester mit Hydrazinhydrat in einem geeigneten Lösungsmittel erhitzt, um das Pyridazon

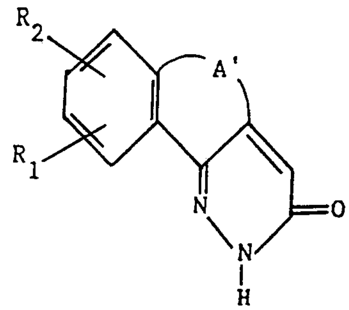

(b)

worin A' wie oben definiert ist zu bilden,

man das Pyridazon mit einem Überschuß an Phosphoroxychlorid oder -oxybromid unter Rückfluß behandelt, um das entsprechende Halogenderivat

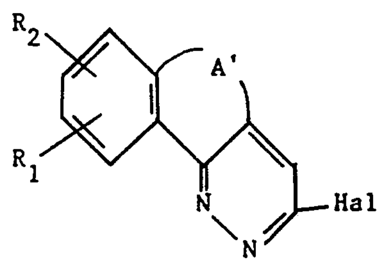

(c)

Hal = Cl, Br zu bilden,

man das Halogenderivat durch Erhitzen mit einem B-$R_3$-Derivat, worin B für OH, SH oder $NH_2$ steht, gegebenenfalls in Gegenwart eines Reaktionsaktivators substituiert, um die entsprechende Verbindung (I) zu bilden;

man gegebenenfalls die Verbindung (I) nach einem bekannten Verfahren in das Salz überführt.

5. Verfahren zur Herstellung von Verbindungen der Formel (I), worin X für $(CH_2)_n$ steht und $R_1$ in der para-Position von X nicht Wasserstoff ist, während $R_2$ = H, dadurch gekennzeichnet, daß

man das Chlorderivat (c), worin $R_1$ und $R_2$ Wasserstoff darstellen, nitriert, um die Verbindung, in der $R_1$ für $NO_2$ steht, zu erhalten;

man das Chlorderivat durch Erhitzen mit einem B-$R_3$-Derivat, wie in Anspruch 4 ausgeführt, substituiert;

man gegebenenfalls das Nitroderivat durch katalytische Reduktion in das entsprechende Aminoderivat überführt;

man gegebenenfalls die Aminogruppe mittels an sich bekannter Verfahren in andere Substituenten überführt;

man gegebenenfalls die so erhaltene Verbindung (I) in das Salz überführt.

6. Verfahren zur Herstellung von Verbindungen der Formel (I), worin X für $(CH_2)_n$ steht und $R_1$ in der para-Position von X nicht Wasserstoff ist, während $R_2$ = H, dadurch gekennzeichnet, daß:

man das Chlorderivat (c), worin $R_1$ und $R_2$ Wasserstoff darstellen, nitriert, um die Verbindung, in der $R_1$ für $NO_2$ steht, zu erhalten;

man dieses Chlorderivat (c) durch katalytische Reduktion in das entsprechende Aminoderivat überführt;

man gegebenenfalls die Aminogruppe mittels an sich bekannter Reaktionen in andere Substituenten überführt;

man das Chlorderivat durch Erhitzen mit einem Derivat B-$R_3$, wie in Anspruch 4 ausgeführt, substituiert;

man gegebenenfalls die so erhaltene Verbindung (I) in das Salz überführt.

7. Verfahren zur Herstellung von Verbindungen der Formel (I), worin X für $(CH_2)_n$ steht und worin Y ein Schwefelatom darstellt, dadurch gekennzeichnet, daß:

man das Pyridazon (b) durch Erhitzen mit einem Überschuß an Phosphorpentasulfid in Gegenwart von Natriumbicarbonat in einem geeigneten Lösungsmittel in das entsprechende Thion überführt;

man das Thion durch Umsetzung eines Halogenids Hal-$R_3$, worin Hal ein Halogen, ausgewählt aus Chlor oder Brom, darstellt, in einem Alkohol und in Gegenwart des entsprechenden Natriumalkoholats oder in Dimethylformamid substituiert;

man gegebenenfalls die so erhaltene Verbindung (I) in das Salz überführt.

8. Verfahren zur Herstellung von Verbindungen der Formel (I), worin X eine Vinylengruppe darstellt, dadurch gekennzeichnet, daß:

man das Chlorderivat (c), worin A' für $—(CH_2)_2—$ steht, mit einem Überschuß des Derivats $BR_3$ in Gegenwart von Ammoniumchlorid auf 120—150°C erhitzt;

man die Verbindung (I), worin X eine Vinylengruppe darstellt, von der Verbindung (I), worin X eine Ethylengruppe darstellt und die sich gleichzeitig gebildet hat, abtrennt;

man gegebenenfalls die so isolierte Verbindung (I) in das Salz überführt.

9. Verfahren zur Herstellung von Verbindungen der Formel (I), worin X die Gruppe Methylvinylen ist, $R_1$, $R_2$, Y und $R_3$ wie in Anspruch 1 definiert sind, dadurch gekennzeichnet, daß es darin besteht, daß:

das Pyridazon der Formel (b), worin A' eine Methylethylengruppe darstellt, mit Phosphoroxychlorid behandelt wird, um die Verbindung der Formel (c) zu erhalten, in der A' eine Methylvinylengruppe darstellt;

das erhaltene Chlorderivat durch Erhitzen mit einem Derivat B-$R_3$, worin B für OH, SH oder $NH_2$ steht, substituiert wird, gegebenenfalls in Gegenwart eines Reaktionsaktivators, um die entsprechende Verbindung (I) zu bilden;

gegebenenfalls die Verbindung (I) nach einem bekannten Verfahren in das Salz übergeführt wird.

10. Verfahren zur Herstellung von Verbindungen der Formel (I), worin

$R_3$ eine Gruppe

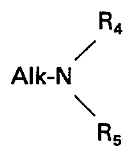 darstellt,

in der $NR_4R_5$ eine 2-Oxo-morpholinogruppe bezeichnet, dadurch gekennzeichnet, daß es darin besteht, daß die entsprechenden Verbindungen der Formel (I), in denen $NR_4R_5$ eine Gruppe $NH—CH_2—CH_2—OH$ darstellt, durch Substitution des Stickstoffs mit Ethylchloracetat und Zyklisierung des so erhaltenen Hydroxyesters behandelt werden.

11. Verfahren zur Herstellung von pharmazeutischen Zusammensetzungen, dadurch gekennzeichnet, daß es darin besteht, eine wirksame Menge einer Verbindung nach einem der Ansprüche 1 bis 3 in einen geeigneten Exzipienten einzuarbeiten.

**Claims for the Contracting States: BE CH DE FR GB IT LI LU NL SE**

1. Tricyclic compounds of the general formula:

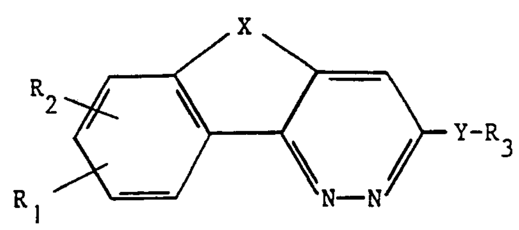

(I)

in which:

X represents a group $(CH_2)_n$, n representing an integer equal to 2, 3 or 4, or alternatively a vinylene group or a methylvinylene group;

$R_1$ and $R_2$, considered independently, represent hydrogen or a substituent occupying one of the free positions of the benzene ring and selected from the group comprising halogens, a lower alkyl group, a lower alkoxy group, a hydroxyl group, a thiol group, a nitro group, an amino group;

Y represents oxygen, sulfur or a group —NH—;
$R_3$ represents:
a group

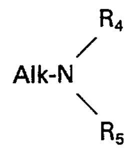 ,

in which:

Alk represents a linear or branched alkylene group having from 2 to 5 carbon atoms;

$R_4$ and $R_5$ each independently represent hydrogen, a lower alkyl group or a lower hydroxyalkyl group, or alternatively $R_4$ and $R_5$ form, with the nitrogen atom to which they are bonded, a group selected from the pyrrolidin-1-yl, piperidino, morpholino, piperazin-1-yl or 2-oxomorpholino groups;

a group

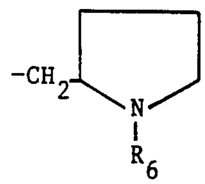

in which $R_6$ represents a lower alkyl group having 1 to 4 carbon atoms;

a group

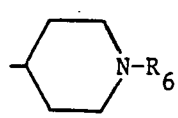

in which $R_6$ is as defined above;

and their salts with pharmaceutically acceptable mineral or organic acids.

2. Compounds according to claim 1, in which X represents a group $(CH_2)_n$ in which n is equal to 2, 3 or 4.

3. Compounds according to claim 1, in which X represents a vinylene or methylvinylene group.

4. Process for the preparation of the compounds of formula (I) in which X represents a group $(CH_2)_n$ n = 2, 3 or 4, characterized in that:

a benzocycloalkan-1-one is treated under the action of heat, at between 60 and 150°C, with ethyl glyoxylate or glyoxylic acid to form the hydroxyester or hydroxyacid:

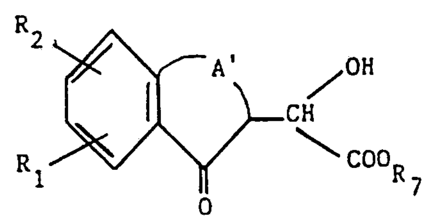

(a)

in which A' represents $(CH_2)_n$ and $R_7$ is H or $C_2H_5$

this hydroxyester is heated with hydrazine hydrate in a suitable solvent to form the pyridazone:

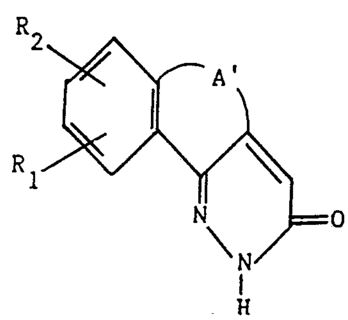

(b)

in which A' is as defined above

the pyridazone is treated under reflux with an excess of phosphorus oxychloride or oxybromide to form the corresponding halogenated derivative:

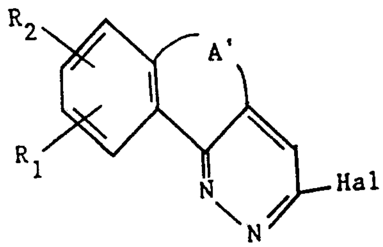

(c)

Hal = Cl, Br

the halogenated derivative is substituted by heating with a derivative B-$R_3$, in which B represents OH, SH or $NH_2$, if appropriate in the presence of a reaction activator, to form the corresponding compound (I); and

if appropriate, the compound (I) is converted to a salt by a known process.

5. Process for the preparation of the compounds of the formula (I) in which X is $(CH_2)_n$ and $R_1$ in the para position relative to X is other than hydrogen, whereas $R_2$ = H, wherein:

the chlorinated derivative (c) in which $R_1$ and $R_2$ represent hydrogen is nitrated to give the compound in which $R_1$ is $NO_2$;

the chlorinated derivative is substituted by heating with a derivative B-$R_3$ as indicated above in claim 4;

if appropriate, the nitro derivative is converted to the corresponding aminated derivative by catalytic reduction;

if appropriate, the amino group is converted to other substituents by reactions known per se; and

if appropriate, the resulting compound (I) is converted to a salt.

6. Process for the preparation of the compounds of the formula (I) in which X = $(CH_2)_n$ and $R_1$ in the para position relative to X is other than hydrogen, whereas $R_2$ = H, characterized in that:

the chlorinated derivative (c) in which $R_1$ and $R_2$ represent hydrogen is nitrated to give the compound in which $R_1$ is $NO_2$;

this chlorinated derivative (c) is converted to the corresponding aminated derivative by catalytic reduction;

if appropriate, the amino group is converted to other substituents by reaction known per se;

the chlorinated derivative is substituted by heating with a derivative B-$R_3$ as indicated in claim 4;

if appropriate, the resulting compound (I) is converted to a salt.

7. Process for the preparation of the compounds of the formula (I) in which X is $(CH_2)_n$ and Y represents a sulfur atom, characterized in that:

the pyridazone (b) is converted to the corresponding thione by heating with an excess of phosphorus pentasulfide in the presence of sodium bicarbonate, in a suitable solvent;

the thione is substituted by reaction with a halide Hal-$R_3$, in which Hal represents a halogen selected from the group comprising chlorine and bromine, in an alcohol and in the presence of the corresponding sodium alcoholate, or in dimethylformamide;

if appropriate, the resulting compound (I) is converted to a salt.

8. Process for the preparation of the compounds (I) in which X is a vinylene group, characterized in that:

the chlorinated derivative (c) in which A' represents $-(CH_2)_2-$ is heated at 120—150°C with an excess of the derivative $BR_3$, in the presence of ammonium chloride;

the compound (I) in which X represents a vinylene group is separated from the compound (I) in which A' represents an ethylene group, which has been formed in conjunction with it;

if appropriate, the compound (I) isolated in this way is converted to a salt.

9. Process for the preparation of the compounds of formula (I) in which X is the methylvinylene group, $R_1$, $R_2$, Y and $R_3$ are as defined in claim 1, characterized in that it consists in:

treating the pyridazone of formula (b) in which A' represents a methylethylene group with phosphorus oxychloride to obtain the compound of formula (c) in which A' is a methylvinylene group;

substituting the chlorinated derivative obtained by heating with a B-$R_3$ derivative in which B represents OH, SH or $NH_2$; if appropriate in the presence of a reaction activating agent in order to form the corresponding compound (I);

if appropriate, salifying the compound (I) according to a known process.

10. Process for the preparation of the compounds of formula (I) in which:

$R_3$ denotes a group

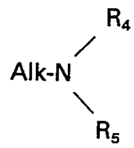

in which $NR_4R_5$ designates a 2-oxo-morpholino group, characterized in that it consists in treating the corresponding compounds of formula (I) in which $NR_4R_5$ denotes a NH—$CH_2$—$CH_2$—OH group by substitution of nitrogen with ethyl chloroacetate and cyclizing of the resulting hydroxyester.

11. Pharmaceutical compositions containing, as the active principle, a compound of the formula (I) in combination with a pharmaceutically acceptable vehicle.

**Claims for the Contracting States: AT ES**

1. Process for obtaining tricyclic compounds of the general formula:

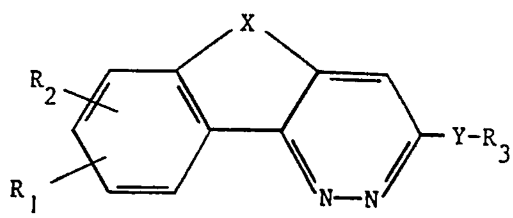

(I)

in which:

X represents a group $(CH_2)_n$, n representing an integer equal to 2, 3 or 4,

$R_1$ and $R_2$, considered independently, represent hydrogen or a substituent occupying one of the free positions of the benzene ring and selected from the group comprising halogens, a lower alkyl group, a lower alkoxy group, a hydroxyl group, a thiol group, a nitro group, an amino group;

Y represents oxygen, sulfur or a group —NH—;

$R_3$ represents:

a group

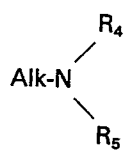

,

in which:

Alk represents a linear or branched alkylene group having from 2 to 5 carbon atoms;

$R_4$ and $R_5$ each independently represent hydrogen, a lower alkyl group or a lower hydroxyalkyl group, or alternatively $R_4$ and $R_5$ form, with the nitrogen atom to which they are bonded, a group selected from the pyrrolidin-1-yl, piperidino, morpholino, piperazin-1-yl or 2-oxomorpholino groups;

a group

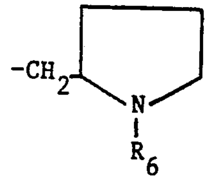

in which $R_6$ represents a lower alkyl group having 1 to 4 carbon atoms;

a group

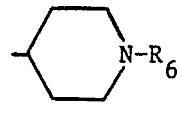

in which $R_6$ is as defined above;
and their salts with pharmaceutically acceptable mineral or organic acids, characterized in that:

a benzocycloalkan-1-one is treated under the action of heat, at between 60 and 150°C, with ethyl glyoxylate or glyoxylic acid to form the hydroxyester or hydroxyacid:

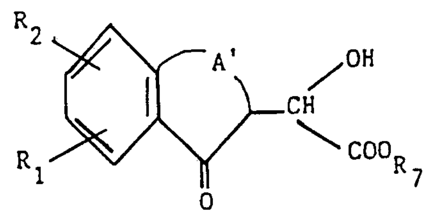

(a)

in which A' represents $(CH_2)_n$ and $R_7$ is H or $C_2H_5$

this hydroxyester is heated with hydrazine hydrate in a suitable solvent to form the pyridazone:

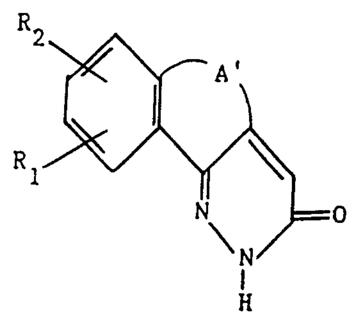

(b)

in which A' is as defined above

the pyridazone is treated under reflux with an excess of phosphorus oxychloride or oxybromide to form the corresponding halogenated derivative:

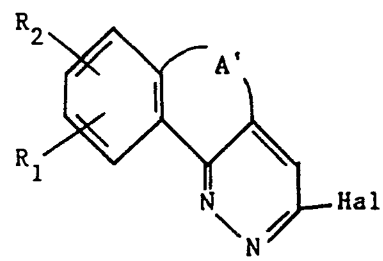

(c)

Hal = Cl, Br

the halogenated derivative is substituted by heating with a derivative B-$R_3$, in which B represents OH, SH or $NH_2$, if appropriate in the presence of a reaction activator, to form the corresponding compound (I); and

if appropriate, the compound (I) is converted to a salt by a known process.

2. Process according to claim 1, for the preparation of the compounds of the formula (I) in which X is $(CH_2)_n$ and $R_1$ in the para position relative to X is other than hydrogen, whereas $R_2$ = H, characterized in that:

the chlorinated derivative (c) in which $R_1$ and $R_2$ represent hydrogen is nitrated to give the compound in which $R_1$ is $NO_2$;

the chlorinated derivative is substituted by heating with a derivative B-$R_3$ as indicated above in claim 1;

if appropriate, the nitro derivative is converted to the corresponding aminated derivative by catalytic reduction;

if appropriate, the amino group is converted to other substituents by reactions known per se; and

if appropriate, the resulting compound (I) is converted to a salt.

3. Process according to claim 1, for the preparation of the compounds of the formula (I) in which X = $(CH_2)_n$ and $R_1$ in the para position relative to X is other than hydrogen, whereas $R_2$ = H, characterized in that:

the chlorinated derivative (c) in which $R_1$ and $R_2$ represent hydrogen is nitrated to give the compound in which $R_1$ is $NO_2$;

this chlorinated derivative (c) is converted to the corresponding aminated derivative by catalytic reduction;

if appropriate, the amino group is converted to other substituents by reaction known per se;
the chlorinated derivative is substituted by heating with a derivative B-$R_3$ as indicated in claim 1;
if appropriate, the resulting compound (I) is converted to a salt.

4. Process according to claim 1, for the preparation of the compounds of the formula (I) in which X is $(CH_2)_n$ and Y represents a sulfur atom, characterized in that:

the pyridazone (b) is converted to the corresponding thione by heating with an excess of phosphorus pentasulfide in the presence of sodium bicarbonate, in a suitable solvent;

the thione is substituted by reaction with a halide Hal-$R_3$, in which Hal represents a halogen selected from the group comprising chlorine and bromine, in an alcohol and in the presence of the corresponding sodium alcoholate, or in dimethylformamide;

if appropriate, the resulting compound (I) is converted to a salt.

5. Process for obtaining tricyclic compounds of the general formula:

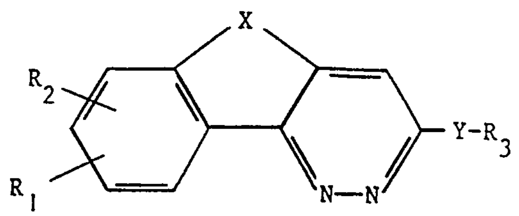

(I)

in which:

X represents a vinylene group;

$R_1$ and $R_2$, considered independently, represent hydrogen or a substituent occupying one of the free positions of the benzene ring and selected from the group comprising halogens, a lower alkyl group, a lower alkoxy group, a hydroxyl group, a thiol group, a nitro group, an amino group;

Y represents oxygen, sulfur or a group —NH—;

$R_3$ represents:

a group

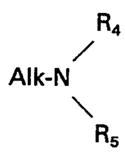

,

in which:

Alk represents a linear or branched alkylene group having from 2 to 5 carbon atoms;

$R_4$ and $R_5$ each independently represent hydrogen, a lower alkyl group or a lower hydroxyalkyl group, or alternatively $R_4$ and $R_5$ form, with the nitrogen atom to which they are bonded, a group selected from the pyrrolidin-1-yl, piperidino, morpholino, piperazin-1-yl or 2-oxomorpholino groups;

a group

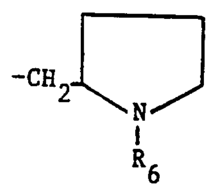

in which $R_6$ represents a lower alkyl group having 1 to 4 carbon atoms;

a group

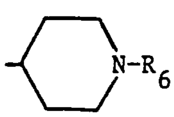

in which $R_6$ is as defined above;

and their salts with pharmaceutically acceptable mineral or organic acids, characterized in that:

the chlorinated derivative (c) in which A' represents $—(CH_2)_2—$ is heated at 120—150°C with an excess of the $BR_3$ derivative, such as defined in claim 1, and in the presence of ammonium chloride;

the compound (I) in which X represents a vinylene group is separated from the compound (I) wherein X represents an ethylene group, which has been formed concomitantly;

if appropriate, the compound (I) thus isolated is converted to a salt.

6. Process for obtaining tricyclic compounds of the general formula:

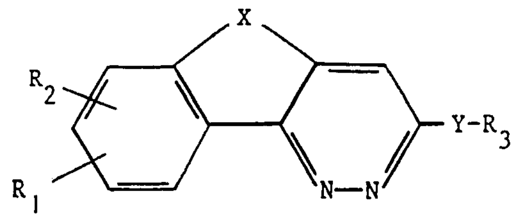

(I)

in which:

X is the methylvinylene group, $R_1$, $R_2$, $R_3$, Y are as defined in claim 1, characterized in that it consists:

in treating the pyridazone of formula (b) wherein A′ represents a methylethylene group, with phosphorous oxychloride to obtain the compound of formula (c) wherein A′ represents a methylvinylene group;

in substituting the resulting chlorinated derivative obtained by heating with a B-$R_3$ derivative wherein B represents OH, SH or $NH_2$; if appropriate in presence of a reaction activating agent to form the corresponding compound (I);

if appropriate, in converting compound (I) into salt according to a known process.

7. Process according to claim 1, for the preparation of the tricyclic compounds of formula (I) in which:

$R_3$ denotes a group

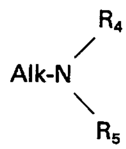

in which $NR_4R_5$ designates a 2-oxo-morpholino group, characterized in that it consists in treating the corresponding compounds of formula (I) in which $NR_4R_5$ denotes a $NH—CH_2—CH_2—OH$ group by substitution of nitrogen with ethyl chloroacetate and cyclizing of the resulting hydroxyester.

8. Use of the tricyclic compounds of the general formula:

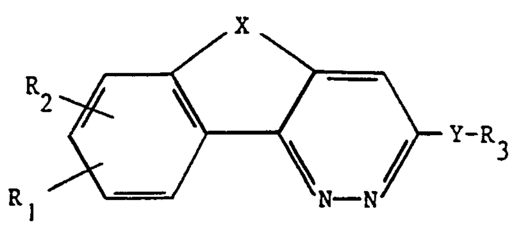

(I)

in which:

X represents a group $(CH_2)_n$, n representing an integer equal to 2, 3 or 4, or alternatively a vinylene group or a methylvinylene group;

$R_1$ and $R_2$, considered independently, represent hydrogen or a substituent occupying one of the free positions of the benzene ring and selected from the group comprising halogens, a lower alkyl group, a lower alkoxy group, a hydroxyl group, a thiol group, a nitro group, an amino group;

Y represents oxygen, sulfur or a group —NH—;

$R_3$ represents:

a group

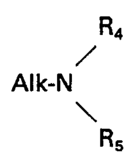

,

in which:

Alk represents a linear or branched alkylene group having from 2 to 5 carbon atoms;

$R_4$ and $R_5$ each independently represent hydrogen, a lower alkyl group or a lower hydroxyalkyl group, or alternatively $R_4$ and $R_5$ form, with the nitrogen atom to which they are bonded, a group selected from the

pyrrolidin-1-yl, piperidino, morpholino, piperazin-1-yl or 2-oxomorpholino groups;

a group

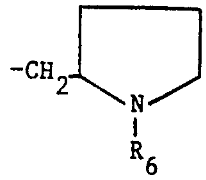

in which $R_6$ represents a lower alkyl group having 1 to 4 carbon atoms;

a group

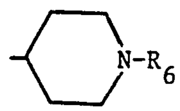

in which $R_6$ is as defined above;
and their salts with pharmaceutically acceptable mineral or organic acids, for the preparation of pharmaceutical compositions, particularly as agonists of the cholinergic receptors.

**Claims for the Contracting State: GR**

1. Tricyclic compounds of the general formula:

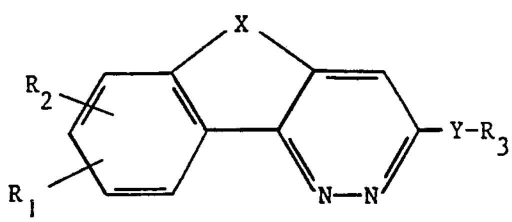

(I)

in which:

X represents a group $(CH_2)_n$, n representing an integer equal to 2, 3 or 4, or alternatively a vinylene group or a methylvinylene group;

$R_1$ and $R_2$, considered independently, represent hydrogen or a substituent occupying one of the free positions of the benzene ring and selected from the group comprising halogens, a lower alkyl group, a lower alkoxy group, a hydroxyl group, a thiol group, a nitro group, an amino group;

Y represents oxygen, sulfur or a group —NH—;

$R_3$ represents:

a group

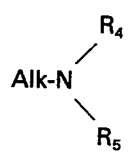

,

in which:

Alk represents a linear or branched alkylene group having from 2 to 5 carbon atoms;

$R_4$ and $R_5$ each independently represent hydrogen, a lower alkyl group or a lower hydroxyalkyl group, or alternatively $R_4$ and $R_5$ form, with the nitrogen atom to which they are bonded, a group selected from the pyrrolidin-1-yl, piperidino, morpholino, piperazin-1-yl or 2-oxomorpholino groups;

a group

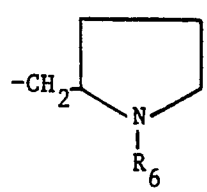

in which $R_6$ represents a lower alkyl group having 1 to 4 carbon atoms;

a group

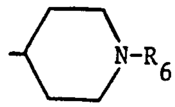

in which $R_6$ is as defined above;

and their salts with pharmaceutically acceptable mineral or organic acids.

2. Compounds according to claim 1, in which X represents a group $(CH_2)_n$ in which n is equal to 2, 3 or 4.

3. Compounds according to claim 1, in which X represents a vinylene or methylvinylene group.

4. Process for the preparation of the compounds of formula (I) in which X represents a group $(CH_2)_n$ n = 2, 3 or 4, characterized in that:

a benzocycloalkan-1-one is treated under the action of heat, at between 60 and 150°C, with ethyl glyoxylate or glyoxylic acid to form the hydroxyester or hydroxyacid:

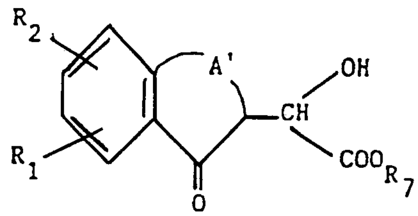

(a)

in which A' represents $(CH_2)_n$ and $R_7$ is H or $C_2H_5$

this hydroxyester is heated with hydrazine hydrate in a suitable solvent to form the pyridazone:

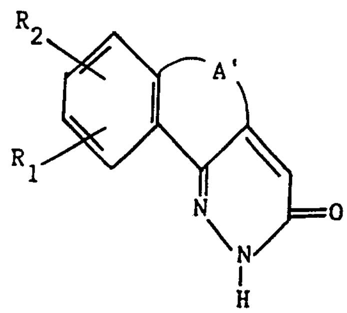

(b)

in which A' is as defined above

the pyridazone is treated under reflux with an excess of phosphorus oxychloride or oxybromide to form the corresponding halogenated derivative:

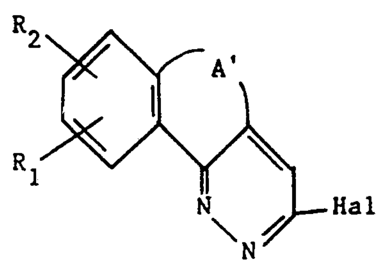

(c)

Hal = Cl, Br

the halogenated derivative is substituted by heating with a derivative B-$R_3$, in which B represents OH, SH or $NH_2$, if appropriate in the presence of a reaction activator, to form the corresponding compound (I); and

if appropriate, the compound (I) is converted to a salt by a known process.

5. Process for the preparation of the compounds of the formula (I) in which X is $(CH_2)_n$ and $R_1$ in the para position relative to X is other than hydrogen, whereas $R_2$ = H, wherein:

the chlorinated derivative (c) in which $R_1$ and $R_2$ represent hydrogen is nitrated to give the compound in which $R_1$ is $NO_2$;

the chlorinated derivative is substituted by heating with a derivative B-$R_3$ as indicated above in claim 4;

if appropriate, the nitro derivative is converted to the corresponding aminated derivative by catalytic reduction;

if appropriate, the amino group is converted to other substituents by reactions known per se; and

if appropriate, the resulting compound (I) is converted to a salt.

6. Process for the preparation of the compounds of the formula (I) in which $X = (CH_2)_n$ and $R_1$ in the para position relative to X is other than hydrogen, whereas $R_2$ = H, characterized in that:

the chlorinated derivative (c) in which $R_1$ and $R_2$ represent hydrogen is nitrated to give the compound in which $R_1$ is $NO_2$;

this chlorinated derivative (c) is converted to the corresponding aminated derivative by catalytic reduction;

if appropriate, the amino group is converted to other substituents by reaction known per se;

the chlorinated derivative is substituted by heating with a derivative B-$R_3$ as indicated in claim 4;

if appropriate, the resulting compound (I) is converted to a salt.

7. Process for the preparation of the compounds of the formula (I) in which X is $(CH_2)_n$ and Y represents a sulfur atom, characterized in that:

the pyridazone (b) is converted to the corresponding thione by heating with an excess of phosphorus pentasulfide in the presence of sodium bicarbonate, in a suitable solvent;

the thione is substituted by reaction with a halide Hal-$R_3$, in which Hal represents a halogen selected from the group comprising chlorine and bromine, in an alcohol and in the presence of the corresponding sodium alcoholate, or in dimethylformamide;

if appropriate, the resulting compound (I) is converted to a salt.

8. Process for the preparation of the compounds (I) in which X is a vinylene group, characterized in that:

the chlorinated derivative (c) in which A′ represents $—(CH_2)_2—$ is heated at 120—150°C with an excess of the derivative $BR_3$, in the presence of ammonium chloride;

the compound (I) in which X represents a vinylene group is separated from the compound (I) in which A′ represents an ethylene group, which has been formed in conjunction with it;

if appropriate, the compound (I) isolated in this way is converted to a salt.

9. Process for the preparation of the compounds of formula (I) in which X is the methylvinylene group, $R_1$, $R_2$, Y and $R_3$ are as defined in claim 1, characterized in that it consists in:

treating the pyridazone of formula (b) in which A′ represents a methylethylene group with phosphorus oxychloride to obtain the compound of formula (c) in which A′ is a methylvinylene group;

substituting the chlorinated derivative obtained by heating with a B-$R_3$ derivative in which B represents OH, SH or $NH_2$; if appropriate in the presence of a reaction activating agent in order to form the corresponding compound (I);

if appropriate, converting the compound (I) to a salt according to a known process.

10. Process for the preparation of the compounds of formula (I) in which:

$R_3$ denotes a group

```
        R4
       /
 Alk-N
       \
        R5
```

in which $NR_4R_5$ designates a 2-oxo-morpholino group, characterized in that it consists in treating the corresponding compounds of formula (I) in which $NR_4R_5$ denotes a $NH—CH_2—CH_2—OH$ group by substitution of nitrogen with ethyl chloroacetate and cyclizing of the resulting hydroxyester.

11. Process for the preparation of pharmaceutical compositions, characterized in that it consists in including an efficient quantity of a compound according to any one of claims 1 to 3 in a suitable vehicle.